(19) Europäisches Patentamt
European Patent Office
Office européen des brevets

(11) Veröffentlichungsnummer: **0 572 450 B1**

# (12) EUROPÄISCHE PATENTSCHRIFT

(45) Veröffentlichungstag der Patentschrift: **26.10.94**

(51) Int. Cl.$^5$: **C09B 29/01**, C09B 29/085, C09B 29/095, C09B 29/30, C09B 29/36, C09B 31/14, C09B 33/10, C09B 33/12

(21) Anmeldenummer: **92904351.1**

(22) Anmeldetag: **10.02.92**

(86) Internationale Anmeldenummer:
**PCT/EP92/00281**

(87) Internationale Veröffentlichungsnummer:
**WO 92/14791 (03.09.92 92/23)**

Die Akte enthält technische Angaben, die nach dem Eingang der Anmeldung eingereicht wurden und die nicht in dieser Patentschrift enthalten sind.

## (54) AZOFARBSTOFFE MIT EINER DIAZOKOMPONENTE AUS DER AMINOBENZOPHENONREIHE SOWIE DISULFONIERTE PYRIDONVERBINDUNGEN.

(30) Priorität: **20.02.91 DE 4105257**

(43) Veröffentlichungstag der Anmeldung:
**08.12.93 Patentblatt 93/49**

(45) Bekanntmachung des Hinweises auf die Patenterteilung:
**26.10.94 Patentblatt 94/43**

(84) Benannte Vertragsstaaten:
**CH DE ES FR GB IT LI**

(56) Entgegenhaltungen:
**EP-A- 0 121 495** **EP-A- 0 126 324**
**EP-A- 0 154 816** **DE-A- 2 004 487**
**FR-A- 2 138 017** **FR-A- 2 214 727**

**Chemical Abstracts, vol. 105, no. 12, September 1986, Columbus, Ohio, US; abstract no. 99348R, T. ARIGA ETa AL.: "water-based inks", Seite 101; siehe Zusammenfassung**

(73) Patentinhaber: **BASF Aktiengesellschaft**
**Carl-Bosch-Strasse 38**
**D-67063 Ludwigshafen (DE)**

(72) Erfinder: **LAMM, Gunther**
**Heinrich-Heine-Strasse 7**
**D-6733 Hassloch (DE)**
Erfinder: **REICHELT, Helmut**
**Johann-Gottlieb-Fichte-Strasse 56**
**D-6730 Neustadt (DE)**
Erfinder: **SCHAFFER, Ortwin**
**Luitpoldstrasse 119**
**D-6700 Ludwigshafen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Benzophenonazofarbstoffe der Formel I

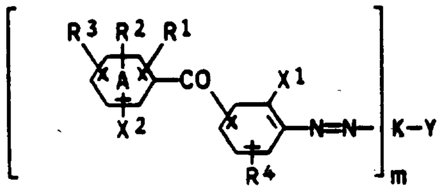

(I),

in der
der Ring A benzoanelliert sein und ein benzoanellierter Ring A durch $C_2H_4$ verbrückt sein kann,

m 1 oder 2,

K den Rest eines 6-Hydroxypyrid-2-onderivats, das in Ringposition 3 unsubstituiert oder durch Carbamoyl, $C_2$-$C_5$-Alkanoyl, Hydroxysulfonylmethyl oder Hydroxysulfonyl substituiert ist, den Rest einer Kupplungskomponente aus der Phenylazopyridon-, Imidazolopyridin-, Aminopyrazol-, Hydroxypyrazol-, Aminothiazol-, Pyrimidin-, Chinolon- oder Anilinreihe oder den Rest einer Kupplungskomponente der Formel IIm

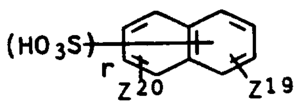

(IIm),

in der

$Z^{19}$ für Amino, Phenylamino, $C_1$-$C_4$-Alkanoylamino oder Benzoylamino,

$Z^{20}$ für Wasserstoff oder Hydroxy und

r für 1 stehen,

Y wenn m die Bedeutung von 1 besitzt, Wasserstoff oder den Rest -N = N-Q, worin Q für den Rest einer Diazo- oder Kupplungskomponente steht, oder, wenn m die Bedeutung von 2 besitzt, Wasserstoff,

einer der beiden Reste $X^1$ und $X^2$ Wasserstoff und der andere Hydroxysulfonyl,

$R^1$, $R^2$ und $R^3$ gleich oder verschieden sind und unabhängig voneinander jeweils Wasserstoff,- Halogen, $C_1$-$C_{12}$-Alkyl, Cyclohexyl, Phenyl, 2-Hydroxyethylsulfonyl oder $C_1$-$C_4$-Alkoxy oder, wenn m für 1 und Y für Wasserstoff stehen, einer dieser Substituenten auch den Rest der Formel

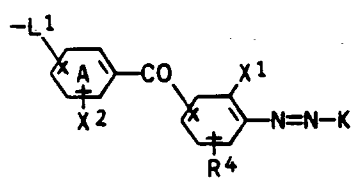

,

worin $L^1$ für eine chemische Bindung, $C_1$-$C_4$-Alkylen, Sauerstoff oder einen Rest der Formel $O$-$CH_2$, $O$-$CH_2CH_2$-$O$, $O$-$CH_2CH_2CH_2$-$O$ oder $O$-$CH(CH_3)CH_2$-$O$ steht und $X^1$, $X^2$, $R^4$, K und der Ring A jeweils die obengenannte Bedeutung besitzen, und

$R^4$ Wasserstoff, Halogen oder $C_1$-$C_4$-Alkoxy bedeuten,

neue Pyridonverbindungen, ein Verfahren zur Herstellung von disulfonierten Pyridonverbindungen sowie die Verwendung der neuen Farbstoffe zum Färben von natürlichen oder synthetischen Substraten.

Die neuen Benzophenonazofarbstoffe der Formel I sind in Form der freien Säure angegeben. Selbstverständlich werden jedoch auch ihre Salze mit umfaßt.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch Hydroxylgruppen substituiert und/oder durch Sauerstoffatome unterbrochen sein kann.

Aus der EP-A-302 401 sowie der älteren Patentanmeldung EP-A-413 229 sind Benzophenonazofarbstoffe bekannt, die jedoch keine wasserlöslich machende Gruppe im Molekül aufweisen.

In der DE-A-2 223 622 ist ein Farbstoff beschrieben, der 3-Aminobenzophenon-4-sulfonsäure als Diazokomponente und 1-Methyl-6-hydroxy-3,4-trimethylenpyrid-2-on als Kupplungskomponente aufweist.

Weiterhin beschreibt die DE-A-3 316 887 einen Azofarbstoff, dessen Kupplungskomponente 1-Hydroxysulfonylbenzyl-3-hydroxysulfonyl-4-methyl-6-hydroxypyrid-2-on ist.

Aus der EP-A-154 816 sind Azofarbstoffe bekannt, die 4-Aminobenzophenon als Diazokomponente und Diaminopyridine als Kupplungskomponente aufweisen.

Schließlich beschreibt die EP-A-126 324 einen Phenylazofabstoff, der ein sulfoniertes N-Benzylpyridon als Kupplungskomponente besitzt.

Aufgabe der vorliegenden Erfindung war es nun, neue Benzophenonazofarbstoffe bereitzustellen, die über mindestens eine Hydroxysulfonylgruppe im Molekül verfügen und die vorteilhafte anwendungstechnische Eigenschaften aufweisen.

Demgemäß wurden die eingangs näher bezeichneten Benzophenonazofarbstoffe der Formel I gefunden.

Alle in der obengenannten Formel I auftretenden Alkyl- und Alkylengruppen können sowohl geradkettig als auch verzweigt sein.

Wenn in den Formeln der erfindungsgemäßen Farbstoffe substituierte Phenylgruppen auftreten, so weisen sie in der Regel 1 bis 3 Substituenten auf. Als Substituenten können z.B. $C_1$-$C_4$-Alkyl, $C_1$-$C_4$-Alkoxy, Halogen oder Hydroxysulfonyl in Betracht kommen.

Kupplungskomponenten KH aus der Pyridon- oder Phenylazopyridonreihe gehorchen z.B. der Formel IIc

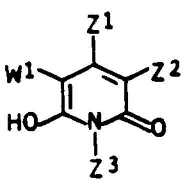

(IIc),

in der

W¹ Wasserstoff oder einen Rest der Formel

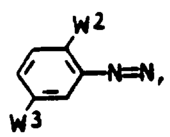

worin

- $W^2$ und $W^3$ gleich oder verschieden sind und unabhängig voneinander jeweils für Wasserstoff, Methyl, Ethyl oder Methoxy stehen,
- $Z^1$ Wasserstoff, $C_1$-$C_4$-Alkyl oder Phenyl,
- $Z^2$ Wasserstoff, Carbamoyl, $C_2$-$C_5$-Alkanoyl, Hydroxysulfonylmethyl oder Hydroxysulfonyl und
- $Z^3$ Wasserstoff oder $C_1$-$C_8$-Alkyl, das gegebenenfalls durch Phenyl oder Hydroxysulfonylphenyl substituiert ist und durch 1 bis 3 Sauerstoffatome in Etherfunktion unterbrochen sein kann, bedeuten.

Kupplungskomponenten KH aus der Imidazolopyridinreihe gehorchen z.B. der Formel IIe

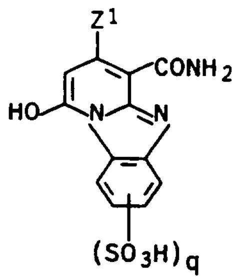

(IIe),

in der q 0 oder 1 bedeutet und $Z^1$ die obengenannte Bedeutung besitzt.

Kupplungskomponenten KH aus der Aminopyrazol- oder Pyrazolonreihe gehorchen z.B. der Formel IIf

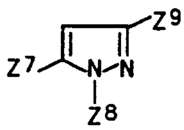

(IIf),

in der

$Z^7$ Amino oder Hydroxy,

$Z^8$ gegebenenfalls durch Phenyl oder Hydroxysulfonylphenyl substituiertes $C_1$-$C_4$-Alkyl oder gegebenenfalls substituiertes Phenyl und

$Z^9$ Wasserstoff, $C_1$-$C_4$-Alkyl, Carboxyl oder $C_1$-$C_4$-Alkoxycarbonyl bedeuten.

Kupplungskomponenten KH aus der Aminothiazolreihe gehorchen z.B. der Formel IIg

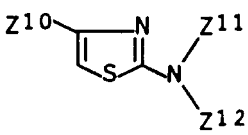

(IIg),

in der

$Z^{10}$ $C_1$-$C_4$-Alkyl, gegebenenfalls substituiertes Phenyl oder Thienyl und

$Z^{11}$ und $Z^{12}$ gleich oder verschieden sind und unabhängig voneinander jeweils $C_1$-$C_8$-Alkyl, das gegebenenfalls durch 1 bis 3 Sauerstoffatome in Etherfunktion unterbrochen ist, bedeuten.

Kupplungskomponenten KH aus der Chinolonreihe gehorchen z.B. der Formel IIh

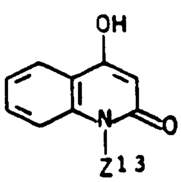

(IIh),

in der

$Z^{13}$ $C_1$-$C_4$-Alkyl bedeutet.

Kupplungskomponenten KH aus der Pyrimidinreihe gehorchen z.B. der Formel IIi oder IIj

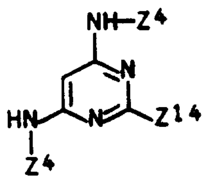

(IIi)

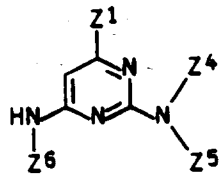

(IIj)

,

worin

$Z^{14}$ $C_1$-$C_4$-Alkyl oder Phenyl bedeutet und

$Z^1$, $Z^4$, $Z^5$ und $Z^6$ jeweils die obengenannte Bedeutung besitzen.

Kupplungskomponenten KH aus der Anilinreihe gehorchen z.B. der Formel III

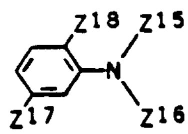

(III),

in der

$Z^{15}$ Wasserstoff, $C_1$-$C_6$-Alkyl, das gegebenenfalls durch Hydroxy, $C_1$-$C_4$-Alkoxy, Cyano, $C_1$-$C_4$-Alkanoyloxy, $C_1$-$C_4$-Alkoxycarbonyloxy, $C_1$-$C_4$-Alkylaminocarbonyloxy, Phenyl, Hydroxysulfonylphenyl, $C_1$-$C_4$Alkoxycarbonyl oder durch Chlor, Hydroxy, $C_1$-$C_4$-Alkoxy oder Phenoxy substituiertes $C_1$-$C_4$-Alkoxycarbonyl substituiert ist,

$Z^{16}$ Wasserstoff oder $C_1$-$C_6$-Alkyl, das durch Phenyl, Hydroxysulfonylphenyl, $C_1$-$C_4$-Alkoxycarbonyl oder durch Chlor, Hydroxy, $C_1$-$C_4$-Alkoxy oder Phenoxy substituiertes $C_1$-$C_4$-Alkoxycarbonyl substituiert sein kann,

$Z^{17}$ Wasserstoff, $C_1$-$C_4$-Alkyl, $C_1$-$C_4$-Alkoxy, Chlor, Brom oder den Rest -NH-CO-Q, wobei Q für $C_1$-$C_4$-Alkyl, das durch $C_1$-$C_4$-Alkoxy, Phenoxy, Cyano, Hydroxy, Chlor oder $C_1$-$C_4$-Alkanoyloxy substituiert sein kann, oder für gegebenenfalls durch $C_1$-$C_4$-Alkoxy substituiertes Phenoxy steht, und

$Z^{18}$ Wasserstoff, $C_1$-$C_4$-Alkyl oder $C_1$-$C_4$-Alkoxy bedeuten.

Wenn Y für den Rest -N = N-Q steht, bedeutet Q den Rest einer Diazo- oder Kupplungskomponente.

Geeignete Diazokomponenten $Q^1$-$NH_2$ leiten sich z.B. aus der Anilinreihe ab. Sie gehorchen z.B. der Formel III

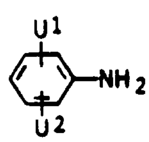

(III),

in der

$U^1$ Wasserstoff, $C_1$-$C_4$-Alkyl oder Hydroxysulfonyl und

$U^2$ Wasserstoff, gegebenenfalls durch Sulfato substituiertes $C_1$-$C_4$-Alkyl, Hydroxysulfonyl, Phenylsulfonyloxy oder 6-Methyl-7-hydroxysulfonylbenzthiazol-2-yl

bedeuten.

Geeignete Kupplungskomponenten $Q^2$-H sind z.B. die oben aufgeführten Verbindungen der Formeln IIc, IIe, IIf, IIg, IIh, IIi, IIj, III oder IIm, wobei Kupplungskomponenten der Formel IIg besonders zu nennen sind.

Reste $R^1$, $R^2$, $R^3$, $Z^1$, $Z^2$, $Z^3$, $Z^4$, $Z^5$, $Z^6$, $Z^8$, $Z^9$, $Z^{10}$, $Z^{11}$, $Z^{12}$, $Z^{13}$, $Z^{14}$, $Z^{15}$, $Z^{16}$, $Z^{17}$, $Z^{18}$, $U^1$ und $U^2$ sind z.B. Methyl, Ethyl, Propyl, Isopropyl, Butyl, Isobutyl oder sec-Butyl.

Reste $R^1$, $R^2$, $R^3$, $Z^3$, $Z^4$, $Z^5$, $Z^6$, $Z^{11}$, $Z^{12}$, $Z^{15}$ und $Z^{16}$ sind weiterhin z.B. Pentyl, Isopentyl, Neopentyl, tert-Pentyl oder Hexyl.

Reste $R^1$, $R^2$, $R^3$, $Z^3$, $Z^4$, $Z^5$, $Z^6$, $Z^{11}$ und $Z^{12}$ sind weiterhin z.B. Heptyl, Octyl, 2-Ethylhexyl oder Isooctyl.

Reste $R^1$, $R^2$, $R^3$, $Z^4$, $Z^5$ und $Z^6$ sind weiterhin z.B. Nonyl, Isononyl, Decyl, Isodecyl, Undecyl oder Dodecyl. (Die obigen Bezeichnungen Isooctyl, Isononyl und Isodecyl sind Trivialbezeichnungen und stammen von den nach der Oxosynthese erhaltenen Alkoholen - vgl. dazu Ullmanns Enzyklopädie der technischen Chemie, 4. Auflage, Band 7, Seiten 215 bis 217, sowie Band 11, Seiten 435 und 436.)
Reste $Z^3$, $Z^4$, $Z^5$, $Z^6$, $Z^8$, $Z^{15}$ und $Z^{16}$ sind weiterhin z.B. Benzyl, Hydroxysulfonylbenzyl, 1- oder 2-Phenylethyl oder 1- oder 2-(Hydroxysulfonylphenyl)ethyl.

Reste $Z^8$ und $Z^{10}$ sind weiterhin z.B. Phenyl, 2-, 3- oder 4-Methylphenyl, 2-, 3- oder 4-Methoxyphenyl, 2-, 3- oder 4-Chlorphenyl, 2,4-Dichlorphenyl, 2-, 3- oder 4-Bromphenyl oder 2-, 3- oder 4-Hydroxysulfonylphenyl.

Reste $Z^3$, $Z^4$, $Z^5$, $Z^6$, $Z^{11}$ und $Z^{12}$ sind weiterhin z.B. 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl oder 3,6,9-Trioxaundecyl.

Reste $Z^4$, $Z^5$ und $Z^6$ sind weiterhin z.B. 2-Hydroxyethyl, 2- oder 3-Hydroxypropyl, 2- oder 4-Hydroxybutyl, 5-Hydroxy-3-oxapentyl, 6-Hydroxy-4-oxahexyl, 8-Hydroxy-4-oxaoctyl, 9-Hydroxy-4,7-dioxanonyl, 2-Phenoxyethyl, 2- oder 3-Phenoxypropyl, 2- oder 4-Phenoxybutyl, 6-Phenoxy-4-oxahexyl, 3,6,9,12-Tetraoxatridecyl, 3,6,9,12-Tetraoxatetradecyl, 2-Formyloxyethyl, 2-Acetyloxyethyl, 2- oder 3-Formyloxypropyl, 2- oder 3-Acetyloxypropyl, 2- oder 4-Formyloxybutyl, 2- oder 4-Acetyloxybutyl, 5-Formyloxy-3-oxapentyl, 5-Acetyloxy-3-oxapentyl, 6-Formyloxy-4-oxaheptyl, 6-Acetyloxy-4-oxyheptyl, 8-Formyloxy-4-oxaoctyl, 8-Acetyloxy-4-oxaoctyl, 9-Formyloxy-4,7-dioxanonyl oder 9-Acetyloxy-4,7-dioxanonyl.

Reste $Z^2$ sind z.B. Acetyl, Propionyl, Butyryl, Isobutyryl oder Pentanoyl.

Reste $Z^9$ sind weiterhin z.B. Methoxycarbonyl, Ethoxycarbonyl, Propoxycarbonyl, Isopropoxycarbonyl, Butoxycarbonyl oder Isobutoxycarbonyl.

$R^{15}$ bedeutet weiterhin z.B. 2-Hydroxyethyl, 2- oder 3-Hydroxypropyl, 4-Hydroxybutyl, 5-Hydroxypentyl, 6-Hydroxyhexyl, 2-Methoxyethyl, 2-Ethoxyethyl, 2-Propoxyethyl, 2-Butoxyethyl, 2-Cyanoethyl, 2-Formyloxyethyl, 2-Acetyloxyethyl, 2-Propionyloxyethyl, 2-Butyryloxyethyl, 4-Acetyloxybutyl, 2-Methoxycarbonyloxyethyl, 2-Methylaminocarbonyloxyethyl, 2-Ethylaminocarbonyloxyethyl, 2-Propylaminocarbonyloxyethyl oder 2-Butylaminocarbonyloxyethyl.

$Z^{15}$ und $Z^{16}$ bedeuten weiterhin Z.B. 2-Methoxycarbonylethyl, 2-Ethoxycarbonylethyl, 2-Propoxycarbonylethyl, 2-Isopropoxycarbonylethyl, 2-Butoxycarbonylethyl, 2-Isobutoxycarbonylethyl, 2-Sec-Butoxycarbonylethyl, 2-(2-Chlorethoxycarbonyl)ethyl, 2-(2-Hydroxyethoxycarbonyl)ethyl, 2-(2-Methoxyethoxycarbonyl)-ethyl, 2-(2-Ethoxyethoxycarbonyl)ethyl, 2-(2-Propoxyethoxycarbonyl)ethyl, 2(2-Isopropxycarbonyl)ethyl, 2-(2-Butoxyethoxycarbonyl)ethyl oder 2-(2-Phenoxyethoxycarbonyl)ethyl.

Wenn $Z^{17}$ für den Rest NH-CO-Q steht, bedeutet Q beispielsweise Methyl, Ethyl, Propyl, Isopropyl, Butyl, Methoxymethyl, Ethoxymethyl, 1- oder 2-Methoxyethyl, 1- oder 2-Ethoxyethyl, Phenoxymethyl, 1- oder 2-Phenoxyethyl, Cyanomethyl, 2-Cyanoethyl, 3-Cyanopropyl, 4-Cyanobutyl, Hydroxymethyl, 1- oder 2-Hydroxyethyl, 2- oder 3-Hydroxypropyl, 4-Hydroxybutyl, Chlormethyl, 2-Chlorethyl, Formyloxymethyl, Acetyloxymethyl, 2-Formyloxyethyl, 2-Acetyloxyethyl, 2-Propionyloxyethyl, 2-Butyryloxyethyl, 3-Formyloxypropyl, 4-Acetyloxybutyl, Phenoxy, 2-Methoxyphenoxy, 4-Methoxyphenoxy, 4-Ethoxyphenoxy oder 4-Isopropoxyphenoxy.

$U^2$ bedeutet weiterhin z.B. 2-Sulfatoethyl oder 2- oder 3-Sulfatopropyl.

Reste $R^1$, $R^2$, $R^3$ und $R^4$ sind weiterhin z.B. Fluor, Chlor, Brom.

Reste $R^1$, $R^2$, $R^3$, $Z^{17}$ und $Z^{18}$ sind weiterhin z.B. Methoxy, Ethoxy, Propoxy, Isopropoxy, Butoxy, Isobutoxy oder sec-Butoxy.

Reste L sind z.B. Methylen, Ethylen, 1,2- oder 1,3-Propylen, Isopropyliden oder 1,2-, 1,3-, 2,3- oder 1,4-Butylen.

Bevorzugt sind Benzophenonazofarbstoffe, die der Formel Ia

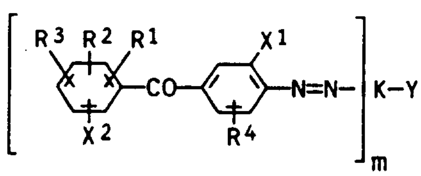

(Ia)

entsprechen, in der m, K, Y, $X^1$, $X^2$, $R^1$, $R^2$, $R^3$ und $R^4$ jeweils die obengenannte Bedeutung besitzen.

Besonders bevorzugt sind Benzophenonazofarbstoffe der Formel I, in der K den Rest eines 6-Hydroxypyrid-2-onderivats, das in Ringposition 3 unsubstituiert oder durch Carbamoyl, $C_2$-$C_5$-Alkanoyl, Hydroxysulfonylmethyl oder Hydroxysulfonyl substituiert ist, den Rest einer Kupplungskomponente aus der Aminopyrazol- oder Pyrimidinreihe oder einen Rest, der sich von der Formel IIm ableitet, bedeutet.

Hervorzuheben sind Benzophenonazofarbstoffe der Formel I, in der K einen Rest der Formel IIc, IIf ($Z^7$ = Amino), IIi, IIj oder IIm bedeutet.

Hervorzuheben sind weiterhin Benzophenonazofarbstoffe der Formel I, in der m 1 und Y Wasserstoff bedeuten.

Hervorzuheben sind weiterhin Benzophenonazofarbstoffe der Formel I, in der $R^1$, $R^2$ und $R^3$ unabhängig voneinander Wasserstoff, Chlor, $C_1$-$C_4$-Alkyl, dabei insbesondere Methyl oder Ethyl, Methoxy oder Ethoxy bedeuten.

Von besonderer Bedeutung sind Benzophenonazofarbstoffe, die der Formel Ib, Ic oder Id

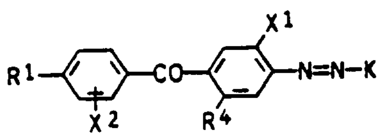
(Ib),

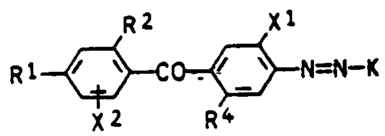
(Ic),

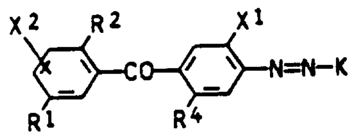
(Id)

entsprechen, worin einer der Reste $X^1$ und $X^2$ Wasserstoff und der andere Hydroxysulfonyl,

- $R^1$ und $R^2$ unabhängig voneinander Chlor, $C_1$-$C_4$-Alkyl, dabei insbesondere Methyl oder Ethyl, Methoxy oder Ethoxy und
- $R^4$ Wasserstoff oder Methoxy bedeuten und
- K jeweils die obengenannte Bedeutung besitzt.

Von besonderer Bedeutung sind auch Benzophenonazofarbstoffe der Formel Ie oder If

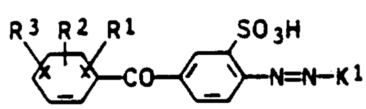
(Ie),

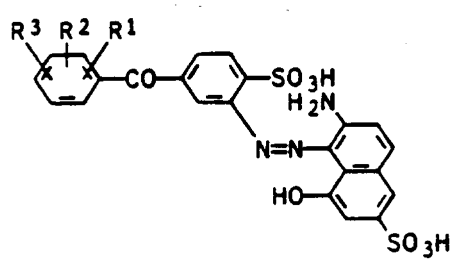
(If),

worin

- $R^1$, $R^2$ und $R^3$ jeweils die obengenannte Bedeutung besitzen und
- $K^1$ 1-Phenyl-3-methyl-5-aminopyrazol-4-yl, 2-Amino-6-hydroxysulfonyl-8-hydroxynaphth-1-yl oder einen Rest der Formel IIo

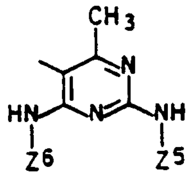

(IIo)

bedeuten, worin $Z^5$ und $Z^6$ jeweils die obengenannte Bedeutung besitzen.

Technisch interessant sind Benzophenonazofarbstoffe der Formel Ig

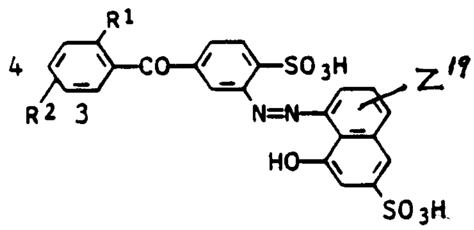

(Ig),

in der

$R^1$ Wasserstoff, $C_1$-$C_3$-Alkyl oder $C_1$-$C_4$-Alkoxy und

$R^2$ $C_1$-$C_{12}$-Alkyl, Benzyl, Phenylethyl oder $C_1$-$C_4$-Alkoxy in Ringposition 3 oder 4 bedeuten, mit der Maßgabe, daß die Gesamtzahl der Kohlenstoffatome in den Resten $R^1$ und $R^2$ 4 bis 12 beträgt.

Die erfindungsgemäßen Aminobenzophenone der Formel I können nach an sich bekannten Methoden erhalten werden.

Beispielsweise diazotiert man ein Aminobenzophenon der Formel III

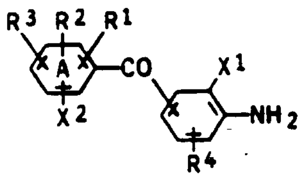

(III),

in der $R^1$, $R^2$, $R^3$, $R^4$, $X^1$, $X^2$ und der Ring A jeweils die obengenannte Bedeutung besitzen, auf übliche Weise und kuppelt es mit einer Kupplungskomponente der Formel IV

H-K-Y (IV),

in der K und Y jeweils die obengenannte Bedeutung besitzen.

Wenn Y in Formel I den Rest $Q^1$-N=N- bedeutet, so können die erfindungsgemäßen Farbstoffe z.B. erhalten werden, indem man zunächst das Diazoniumsalz des Amins der Formel V

$Q^1$-$NH_2$ (V),

in der $Q^1$ die obengenannte Bedeutung besitzt, mit der Kupplungskomponente der Formel IVa

H-K-H (IVa),

in der K die obengenannte Bedeutung besitzt, kuppelt und den resultierenden Monoazofarbstoff der Formel VI

H-K-N=N-$Q^1$ (VI),

in der Q1 und K jeweils die obengenannte Bedeutung besitzen, mit dem Diazoniumsalz des Aminobenzophenons III kuppelt. Es ist aber auch möglich, die beiden Kupplungsreaktionen in ihrer Reihenfolge umzukehren.

Es ist weiterhin möglich, hierbei anstelle des Amins der Formel V ein Aminobenzophenon der Formel III zu verwenden. Man gelangt dann zu Disazofarbstoffen, die zwei verschiedene oder gleiche Reste der Diazokomponente der Formel III aufweisen.

Stammt die Kupplungskomponente der Formel IVa aus der Anilin- oder Naphthalinreihe (Formel III oder IIm) und verfügt sie über eine unsubstituierte Aminogruppe oder deren Vorläufer, so kann der resultieren Monoazofarbstoff der Formel VII

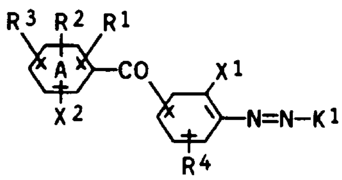

(VII),

in der $K^1$ den Rest einer Kupplungskomponente aus der Anilin- oder Naphthalinreihe, der über eine Aminogruppe verfügt, bedeutet und $R^1$, $R^2$, $R^3$, $R^4$, $X^1$, $X^2$ und der Ring A jeweils die obengenannte Bedeutung besitzen, auch erneut diazotiert und mit einer Kupplungskomponente der Formel IVa gekuppelt werden. Auf diese Weise gelangt man ebenfalls zu Disazofarbstoffen.

Bei den Aminobenzophenonen der Formel III handelt es sich um an sich bekannte Verbindungen. Sie sind z.B. in der älteren europäischen Patentanmeldung Nr. 91112002.0 beschrieben.

Bei den Kupplungskomponenten der Formel IV handelt es sich ebenfalls um an sich bekannte Produkte. Sie sind beispielsweise in D.R. Waring, G. Hallas "The Chemistry and Application of Dyes", Plenum Press, New York, 1990, oder M. Okawara, T. Kitao, T. Hirashima, M. Matsuoka "Organic Colorants", Elsevier, Amsterdam, 1988, oder in der dort genannten Literatur beschrieben.

Die vorliegende Erfindung betrifft weiterhin neue Pyridonverbindungen der Formel II

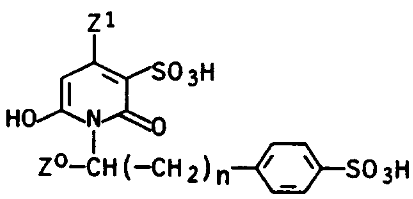

(II),

in der

n 1 oder 2,

$Z^o$ Wasserstoff oder $C_1$-$C_4$-Alkyl und

$Z^1$ Wasserstoff, $C_1$-$C_4$-Alkyl oder Phenyl bedeuten.

Bevorzugt sind Pyridonverbindungen der Formel II, in der n 1, $Z^1$ $C_1$-$C_4$-Alkyl, insbesondere Methyl, und $Z^o$ Wasserstoff oder Methyl, insbesondere Methyl, bedeuten.

Monosulfonsäuren ähnlicher Art sind aus der DE-A 2 117 753 bekannt.

Die Pyridonverbindungen II eignen sich in vorteilhafter Weise als Kupplungskomponenten für die Herstellung von Azofarbstoffen.

Sie können erhalten werden, indem man beispielsweise ein Hydroxypyridon der Formel VIII

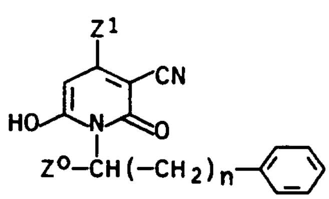

(VIII),

in der n, $Z^o$ und $Z^1$ jeweils die obengenannte Bedeutung besitzen, im Backverfahren oder in einer zweistufigen Reaktion im Eintopfverfahren mit konzentrierter Schwefelsäure, gegebenenfalls in Anwesenheit von Oleum, umsetzt. In der Regel wird dabei die 1. Stufe bei einer Temperatur von 30 bis 70 °C und die 2. Stufe bei einer Temperatur von 120 bis 150 °C durchgeführt.

Es wurde weiterhin gefunden, daß die Herstellung der Pyridonverbindungen der Formel IIa

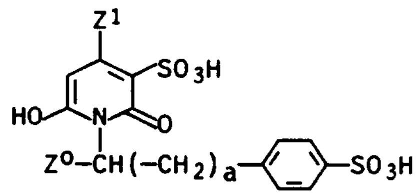

(IIa),

in der

a 0, 1 oder 2,

$Z^o$ Wasserstoff oder $C_1$-$C_4$-Alkyl und

$Z^1$ Wasserstoff, $C_1$-$C_4$-Alkyl oder Phenyl bedeuten,

durch Umsetzung von Pyridonen der Formel IIb

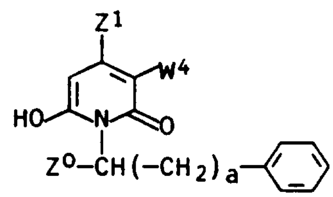

(IIb),

in der $W^4$ Cyano oder Acetyl bedeutet und a, $Z^o$ und $Z^1$ jeweils die obengenannte Bedeutung besitzen, mit Oleum vorteilhaft gelingt, wenn man Oleum verwendet, das 1 bis 2 mol freies Schwefeltrioxid enthält, und man die Umsetzung in einer 1. Stufe 1 bis 5 Stunden bei einer Temperatur von 0 bis 75 °C und anschließend in einer 2. Stufe 2 bis 11 Stunden bei einer Temperatur von 80 bis 135 °C durchführt.

Dabei wird in der 1. Stufe die Sulfonsäuregruppe in den Phenylring und in der 2. Stufe in den Pyridinring eingeführt.

Die Aufarbeitung des Reaktionsgemisches erfolgt nach üblichen Methoden, z.B. durch Ausrühren auf Eiswasser und anschließendes Neutralisieren, z.B. mit Natronlauge.

Als Oleum verwendet man vorteilhaft ein solches Produkt, das 1 bis 2 mol, vorzugsweise 1 bis 1,5 mol, freies Schwefeltrioxid enthält.

Das Molverhältnis Oleum : Pyridon IIb beträgt in der Regel 0,5 : 1 bis 0,55 : 1, vorzugsweise ca. 0,51 : 1.

Es ist auch möglich, mehr Oleum oder Oleum mit einem höheren Gehalt an Schwefeltrioxid zu verwenden, jedoch bringt dies keine weiteren Vorteile. Vielmehr benötigt man bei der anschließenden Aufarbeitung des Reaktionsgemisches deutlich mehr Eiswasser und Natronlauge.

Die erfindungsgemäßen Benzophenonazofarbstoffe der Formel I eignen sich in vorteilhafter Weise zum Färben von natürlichen oder synthetischen Substraten, beispielsweise von Wolle, Leder oder Polyamid. Man erhält Färbungen mit guten Gebrauchsechtheiten.

Sie eignen sich weiterhin für das Ink-Jet-Verfahren.

Die folgenden Beispiele sollen die Erfindung näher erläutern.

Beispiel 1

21,2 g der Diazokomponente der Formel

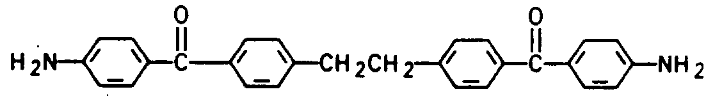

wurden mit 60 ml konzentrierter Salzsäure, 60 ml Eisessig und 0,2 g eines sauer wirksamen Netzmittels verrührt. Dann kühlte man das Gemisch auf 0 °C ab und gab bei 0 bis 6 °C 31 ml 23 gew.-%ige, wäßrige Natriumnitritlösung hinzu. Man erhielt nach kurzer Zeit eine klare Diazoniumsalzlösung, die 2 Stunden bei 0 bis 6 °C nachgerührt wurde. Danach zerstörte man überschüssige salpetrige Säure mit Amidosulfonsäure. Dann versetzte man dieses Gemisch mit 35,6 g der Kupplungskomponente der Formel

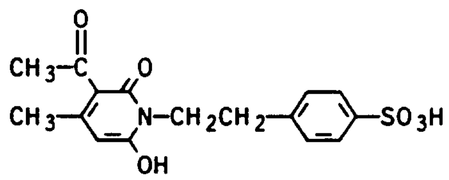

,

die vorher in 250 ml Wasser mit 26 g 50 gew.-%iger Natronlauge gelöst und auf 0 °C abgekühlt wurden.

Das Kupplungsgemisch wurde bei 0 bis 6 °C mit Natronlauge auf einen pH-Wert von 3,5 bis 4 abgepuffert. Der entstandene Farbstoff der Formel

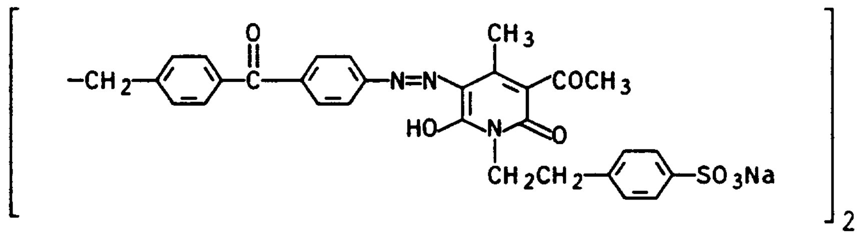

wurde mit Natriumchlorid ausgefällt, dann abgesaugt, getrocknet und gemahlen. Man erhielt 120 g eines gelben Pulvers mit einem Farbstoffgehalt von 50 Gew.-%.

Das Absorptionsmaximum einer Farbstofflösung in einem Gemisch aus 1 g Eisessig und 9 g N,N-Dimethylformamid beträgt 428 nm.

Der Farbstoff färbt Leder, Polyamid und Wolle in zitronengelbem Ton mit guter Naß- und Lichtechtheit.

Beispiel 2

Man arbeitete analog Beispiel 1, ersetzte jedoch die Kupplungskomponente durch 35,7 g der Kupplungskomponente der Formel

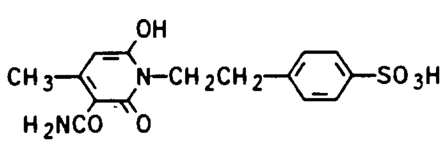

und erhielt 59,5 g des Farbstoffes der Formel

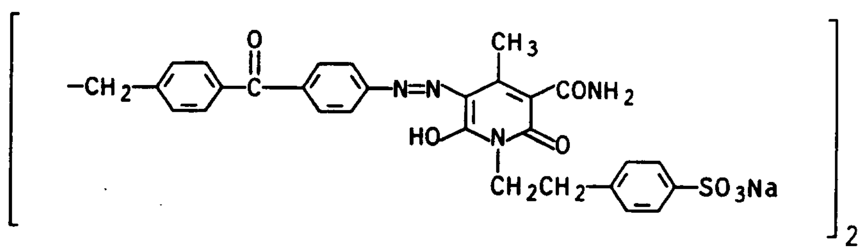

als 50 gew.-%iges gelbes Pulver, dessen Absorptionsmaximum in einer Lösung aus N,N-Dimethylformamid/Eisessig (9:1 g/g) 429 nm beträgt.

Der Farbstoff färbt Wolle und Polyamid in klarem kräftigen zitronengelben Ton mit guten Echtheiten.

Beispiel 3

Man verfuhr analog Beispiel 1, ersetzte aber die Kupplungskomponente durch 20,5 g der Kupplungskomponente der Formel

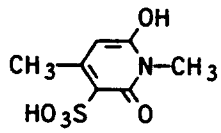

und erhielt 42 g des Farbstoffes der Formel

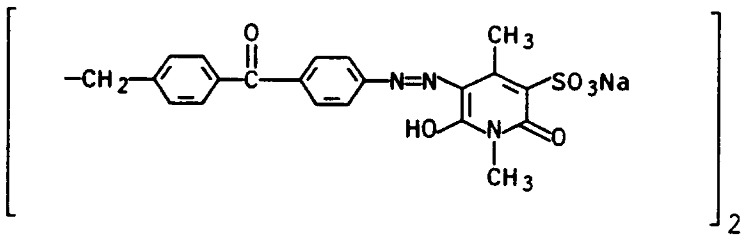

als 90 gew.-%iges Pulver.

Der Farbstoff färbt Polyamid, Wolle und Leder in klarem zitronengelbem, echtem Ton. Die Färbungen weisen eine gute Lichtechtheit auf.

Im Ink-Jet-Verfahren erhält man Drucke mit guten Gebrauchsechtheiten.

Das Absorptionsmaximum in Wasser beträgt 420 nm.

Beispiel 4

Man diazotierte 21,1 g der Diazokomponente der Formel

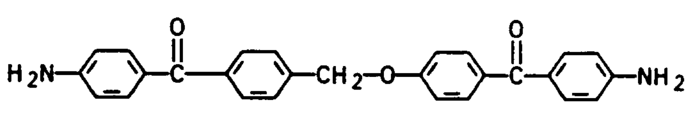

analog zu dem in Beispiel 1 beschriebenen Verfahren und kuppelte in analoger Weise mit 20,5 g 1,4-Dimethyl-6-hydroxy-3-hydroxysulfonylpyrid-2-on. Man erhielt 42 g des Farbstoffes der Formel

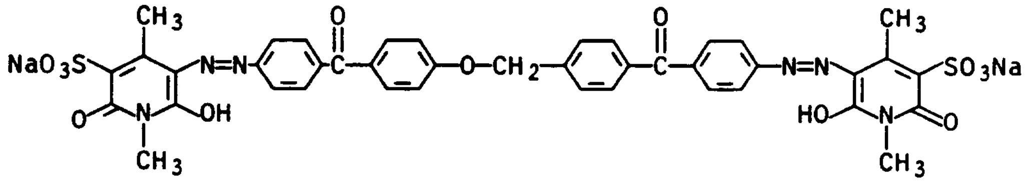

als 90 gew.-%iges Pulver.

Der Farbstoff färbt Leder, Polyamid und Wolle in echtem zitronengelben Ton.

Im Ink-Jet-Verfahren erhält man Drucke mit guten Gebrauchsechtheiten.

Beispiel 5

58 g der Diazokomponente der Formel

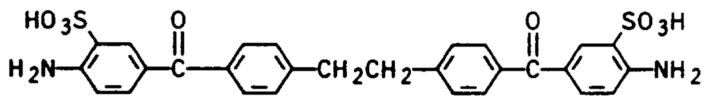

wurden in 500 ml Wasser mit 0,2 g eines sauer wirksamen Netzmittels verrührt. Dann hob man den pH-Wert des Gemisches durch Zugabe von 16,5 g Natronlauge (50 gew.-%ig) auf 8,5 bis 11,5 an und gab danach 62 ml 23 gew.-%ige wäßrige Natriumnitritlösung hinzu. Dieses Gemisch wurde in dem Maße zu 70 ml konzentrierter Salzsäure, die mit Eis verdünnt war, gegeben, daß die Temperatur des Diazoniumsalzgemisches stets unter 8 °C blieb. Man rührte die Diazoniumsalz-Suspension 4 Stunden bei 0 bis 6 °C nach. Dann zerstörte man überschüssige salpetrige Säure wie üblich, gab eine Lösung von 34,6 g 1-Phenyl-3-methyl-5-aminopyrazol in verdünnter Salzsäure hinzu und hob den pH-Wert des Reaktionsgemisches nach und nach auf 2,3 bis 3,1 an. Man ließ über Nacht bei einem pH-Wert von 3 nachrühren. Der entstandene Farbstoff der Formel

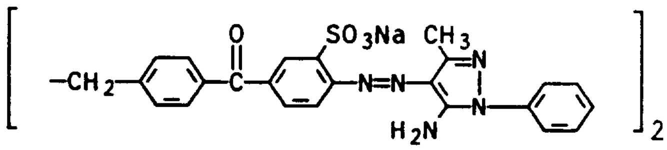

wurde wie üblich isoliert. Man erhielt 116 g eines orangefarbenen 80 gew.-%igen Pulvers. Der Farbstoff färbt Wolle und Polyamid in echtem goldgelben Ton.

Beispiel 6

29,1 g 4-Amino-4'-methylbenzophenon-3-sulfonsäure wurden mit Natronlauge in 200 ml Wasser bei einem pH-Wert von 8 bis 11 gelöst. Dazu gab man 31 ml 23 gew.-%ige wäßrige Natriumnitritlösung. Dann rührte man die Lösung auf ein Gemisch von 32 ml konz. Salzsäure und Eis in dem Maße aus, daß die Temperatur nicht über +8 °C anstieg. Die Suspension wurde 3 Stunden bei 0 bis 5 °C nachgerührt. Dann zerstörte man überschüssige salpetrige Säure mit Amidosulfonsäure und gab danach 17,3 g 1-Phenyl-3-methyl-5-aminopyrazol, gelöst in 300 ml Wasser und 10 ml konzentrierter Salzsäure, hinzu.

Anschließend hob man den pH-Wert des Gemisches mit Natronlauge und wenig Natriumformiat auf 2,6 bis 3,5 an. Die Kupplung war nach ca. 2 Stunden beendet. Zur Kristallisation des Farbstoffes der Formel

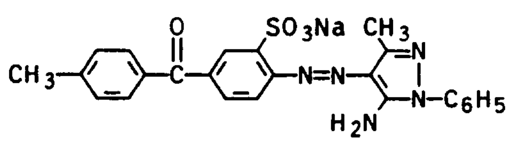

wurde das Reaktionsgemisch angeimpft und über Nacht gerührt. Man isolierte und trocknete wie üblich und erhielt 54 g eines orangefarbenen Pulvers mit einem Farbstoffgehalt von 91 Gew.-% (Restgehalt: Wasser und Natriumchlorid).

Der Farbstoff färbt Polyamid und Wolle in kräftigem goldgelben Ton mit vorzüglicher Lichtechtheit gleichmäßig an. Das Migrationsvermögen ist ausgezeichnet.

Beispiel 7

30,7 g der Diazokomponente 4-Amino-4'-methoxybenzophenon-3'-sulfonsäure wurden analog Beispiel 6 diazotiert. Die erhaltene Suspension des Diazoniumsalzes wurde mit 17,3 g 1-Phenyl-3-methyl-5-aminopyrazol, gelöst in 150 ml Wasser mit 9 ml konzentrierter Salzsäure, versetzt. Dann hob man den pH-Wert des Reaktionsgemisches nach und nach auf 2,6 bis 3,5 an und rührte 2 Stunden bei einem pH-Wert von 3 bis 3,5 nach. Der Farbstoff der Formel

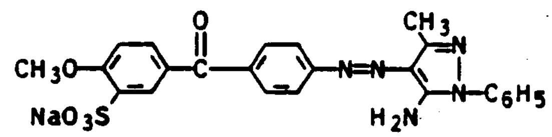

wurde mit Natriumchlorid ausgefällt, wie üblich isoliert, getrocknet und gemahlen. Man erhielt 51,2 g Farbstoff als 75 gew.-%iges gelbes Pulver.

Der Farbstoff hat auf Polyamid und Wolle gutes Ziehvermögen und gleicht insbesondere auf Polyamid Materialunterschiede gut aus. Sein Migrationsverhalten und die Lichtechtheit auf Polyamid sind gut. Polyamid und Wolle werden grünstichig gelb angefärbt.

Beispiel 8

29,1 g 4-Amino-4'-methylbenzophenon-3'-sulfonsäure wurden analog Beispiel 6 diazotiert. Nach Zerstören von überschüssiger salpetriger Säure vereinigte man die Suspension des Diazoniumsalzes mit einer frisch hergestellten feinen Suspension von 31,9 g 1-Hydroxy-8-aminonaphthalin-3,6-disulfonsäure, die mit Salzsäure auf einen pH-Wert von 0,5 bis 0 angesäuert wurde. Man ließ das Reaktionsgemisch über Nacht bei 5 bis 10 °C rühren und erhielt einen roten Farbstoff der Formel

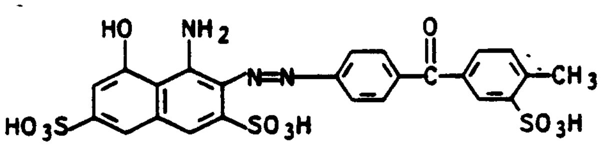

$\lambda_{max}$ (Wasser): 601 nm

Der Farbstoff wurde durch Versetzen des Gemisches mit Natriumchlorid vollständig ausgefällt und wie üblich isoliert. Das so isolierte Produkt wurde mit Natronlauge in 1000 ml Wasser bei einem pH-Wert von 6 bis 9 gelöst. Dann versetzte man die Lösung mit 0,1 Mol diazotiertem 4-Amino-2',5'-dimethylbenzophenon und hielt den pH-Wert des Reaktionsgemisches bei > 6 bis 8. Der entstandene Farbstoff der Formel

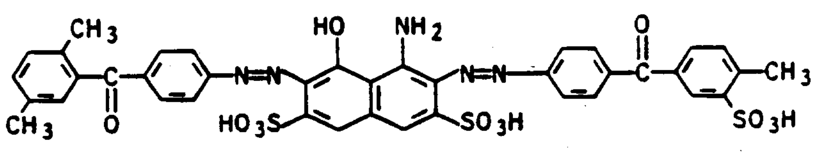

wurde bei pH 6 bis 8 mit Natriumchlorid als Trinatriumsalz ausgefällt und wie üblich isoliert. Man erhielt 120 g eines schwarzen Pulvers, das Leder, Polyamid und Wolle in marinebiauem Ton färbt. Der Farbstoff enthält noch ca. 24 g Natriumchlorid.

Das Absorptionsmaximum des Farbstoffes in Wasser beträgt 604 nm.

In analoger Weise werden die im folgenden aufgeführten Farbstoffe erhalten.

Tabelle 1

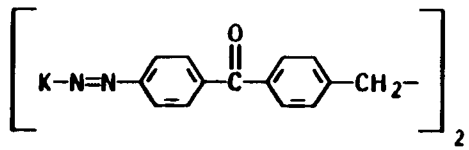

| Bsp. Nr. | K | E | G | Farbton | $\lambda_{max}$ [nm] |
|---|---|---|---|---|---|
| 9 | 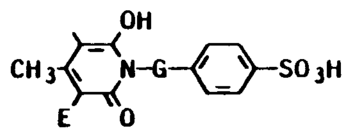 | $CONH_2$ | $CH_2$ | zitronengelb | |
| 10 | 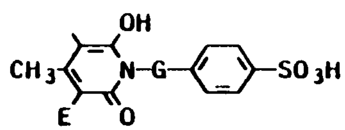 | $COCH_3$ | $CH_2$ | zitronengelb | |
| 11 | 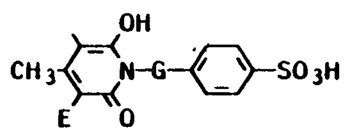 | $COCH_3$ | $CH_2-CH$<br>$CH_3$ | zitronengelb | |
| 12 | 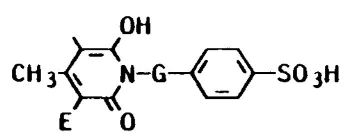 | $COCH_3$ | $CH_2CH_2CH_2$ | zitronengelb | |

Tabelle 1 (Fortsetzung)

| Bsp. Nr. | K | E | G | Farbton | $\lambda_{max}$ [nm] |
|---|---|---|---|---|---|
| 13 | 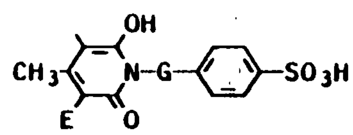 | $CONH_2$ | 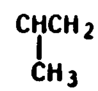 | zitronengelb | |
| 14 | 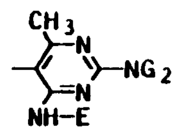 | 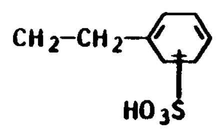 | $C_2H_5$ | gelb | |
| 15 | 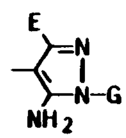 | $CH_3$ | 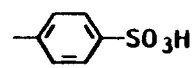 | grünstichig gelb | |
| 16 | 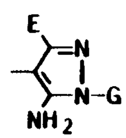 | H | 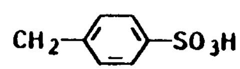 | grünstichig gelb | |

Tabelle 1 (Fortsetzung)

| Bsp. Nr. | K | E | G | Farbton | $\lambda_{max}$ [nm] |
|---|---|---|---|---|---|
| 17 | 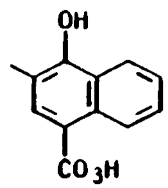 | - | - | scharlach | |
| 18 | 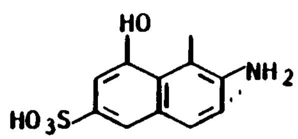 | - | - | rot | 509 (in Wasser) |
| 19 | 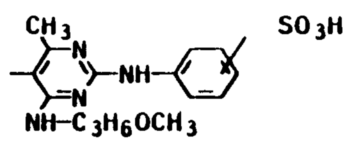 | - | - | gelb | |

Tabelle 2 (Fortsetzung)

| Bsp. Nr. | K | $R^1$ | $R^2$ | $R^3$ | $G^1$ | $G^2$ | Farbton |
|---|---|---|---|---|---|---|---|
| 20 | 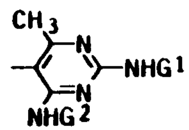 | H | $CH_3$ | H | 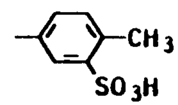 | $C_4H_9(n)$ | gelb |
| 21 | 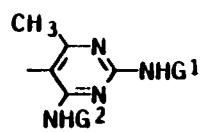 | $CH_3$ | $CH_3$ | H | 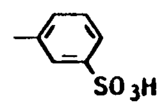 | $C_4H_9(n)$ | gelb |

Tabelle 2 (Fortsetzung)

| Bsp. Nr. | K | $R^1$ | $R^2$ | $R^3$ | $G^1$ | $G^2$ | Farbton |
|---|---|---|---|---|---|---|---|
| 22 | 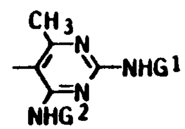 | $CH_3$ | H | $CH_3$ | 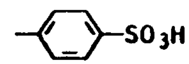 | $C_4H_9(n)$ | gelb |
| 23 | 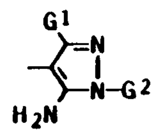 | H | $CH_3$ | H | H | 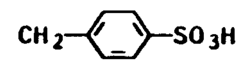 | gelb |
| 24 | 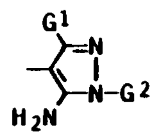 | $CH_3$ | $CH_3$ | H | H | 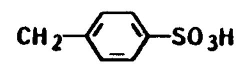 | gelb |
| 25 | 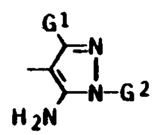 | $CH_3$ | $CH_3$ | H | $CH_3$ | 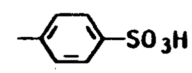 | gelb |
| 26 | 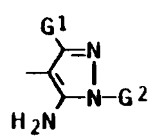 | H | Cl | H | $CH_3$ | $C_6H_5$ | gelb |

Tabelle 2 (Fortsetzung)

| Bsp. Nr. | K | $R^1$ | $R^2$ | $R^3$ | $G^1$ | $G^2$ | Farbton |
|---|---|---|---|---|---|---|---|
| 27 | 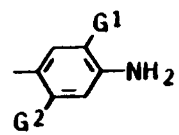 | H | $OCH_3$ | H | $OCH_3$ | $OCH_3$ | orange |
| 28 | 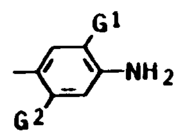 | H | $OCH_3$ | H | $OCH_3$ | $OCH_3$ | orange |
| 29 | 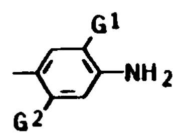 | H | $OCH_3$ | H | $CH_3$ | $CH_3$ | orange |

Tabelle 3

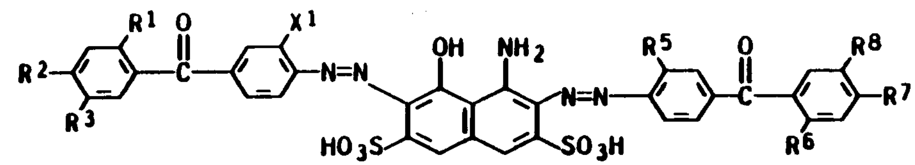

| Bsp. Nr. | $R^1$ | $R^2$ | $R^3$ | $X^1$ | $R^5$ | $R^6$ | $R^7$ | $R^8$ |
|---|---|---|---|---|---|---|---|---|
| 30 | H | $CH_3$ | $CH_3$ | H | $SO_3H$ | H | $OCH_3$ | H |
| 31 | $CH_3$ | $CH_3$ | H | H | $SO_3H$ | H | $OCH_3$ | H |
| 32 | H | $CH_3$ | $CH_3$ | H | $SO_3H$ | H | $CH_3$ | H |
| 33 | H | $CH_3$ | H | H | $SO_3H$ | H | $CH_3$ | H |
| 34 | H | $CH_3$ | $CH_3$ | H | H | H | $OCH_3$ | $SO_3H$ |
| 35 | $CH_3$ | $CH_3$ | H | H | H | H | $OCH_3$ | $SO_3H$ |
| 36 | H | $CH_3$ | $CH_3$ | H | H | H | $CH_3$ | $SO_3H$ |
| 37 | H | $CH_3$ | H | H | H | H | $CH_3$ | $SO_3H$ |
| 38 | $OCH_3$ | H | $CH_3$ | H | H | $CH_3$ | $CH_3$ | $SO_3H$ |
| 39 | H | $CH_3$ | H | $SO_3H$ | H | $OCH_3$ | $CH_3$ | H |
| 40 | H | $OCH_3$ | H | $SO_3H$ | H | H | $OCH_3$ | H |
| 41 | H | $C_2H_5$ | H | H | $SO_3H$ | H | $C_2H_5$ | H |
| 42 | H | $C_2H_5$ | H | H | H | H | $C_2H_5$ | $SO_3H$ |
| 43 | $CH_3$ | $CH_3$ | H | H | H | H | $CH_3$ | $SO_3H$ |
| 44 | H | Cl | H | H | H | H | $CH_3$ | $SO_3H$ |
| 45 | H | $C_2H_5$ | H | H | H | H | $CH_3$ | $SO_3H$ |

Tabelle 4

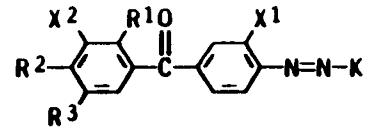

| Bsp. Nr. | $R^1$ | $R^2$ | $R^3$ | $X^1$ | $X^2$ | K | Farbton |
|---|---|---|---|---|---|---|---|
| 46 | H | $CH_3$ | H | $SO_3H$ | H | 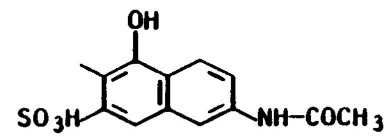 | orange |
| 47 | H | $CH_3$ | $CH_3$ | $SO_3H$ | H | 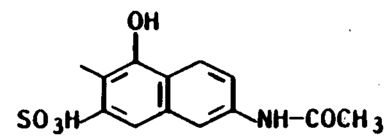 | orange |
| 48 | H | $CH_3$ | $CH_3$ | H | $SO_3H$ | 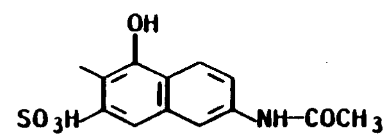 | orange |
| 49 | $CH_3$ | $CH_3$ | H | $SO_3H$ | H | 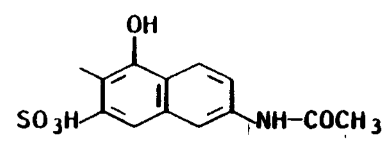 | orange |

Tabelle 4 (Fortsetzung)

| Bsp. Nr. | $R^1$ | $R^2$ | $R^3$ | $X^1$ | $X^2$ | K | Farbton |
|---|---|---|---|---|---|---|---|
| 50 | H | $OCH_3$ | H | $SO_3H$ | H | 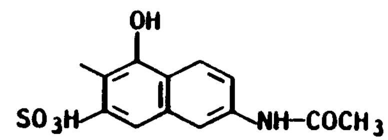 | orange |
| 51 | H | $C_2H_5$ | H | $SO_3H$ | H | 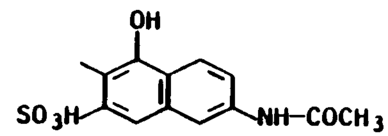 | orange |
| 52 | H | Cl | H | $SO_3H$ | H | 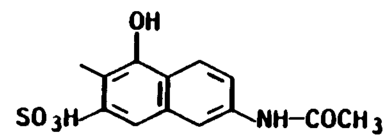 | orange |
| 53 | H | $C_2H_5$ | H | $SO_3H$ | H | 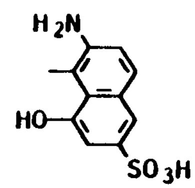 | rot |
| 54 | H | $CH_3$ | H | $SO_3H$ | H | 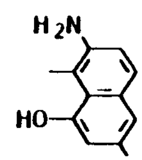 | rot |

Tabelle 4 (Fortsetzung)

| Bsp. Nr. | $R^1$ | $R^2$ | $R^3$ | $X^1$ | $X^2$ | K | Farbton |
|---|---|---|---|---|---|---|---|
| 55 | H | $OCH_3$ | H | $SO_3H$ | H | 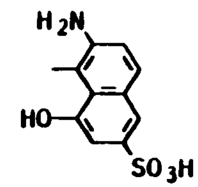 | rot |
| 56 | H | $OCH_3$ | $SO_3H$ | H | H | 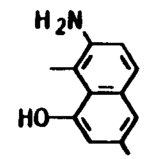 | rot |
| 57 | H | $C_2H_5$ | $SO_3H$ | H | H | 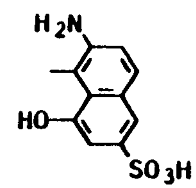 | rot |
| 58 | H | $CH_3$ | $SO_3H$ | $SO_3H$ | H | 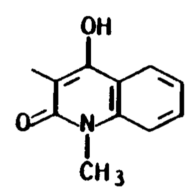 | gelb |

Tabelle 5

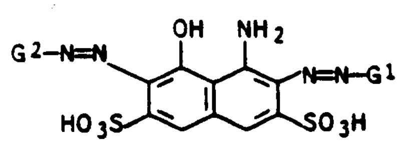

| Bsp. Nr. | $G^1$ | $G^2$ | Farbton |
|---|---|---|---|
| 59 | 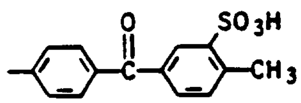 | 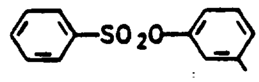 | blau |
| 60 | 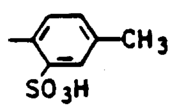 | 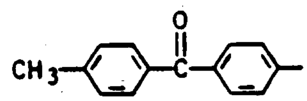 | blau |
| 61 | 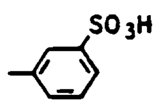 | 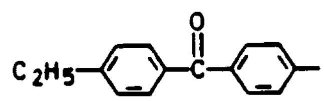 | blau |
| 62 | 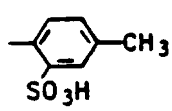 | 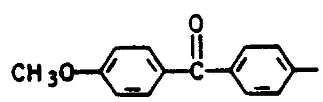 | blau |
| 63 | 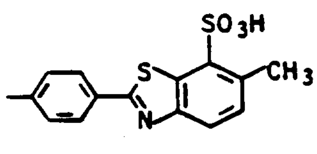 | 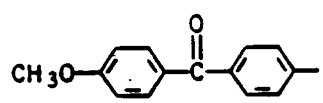 | blau-schwarz |
| 64 | 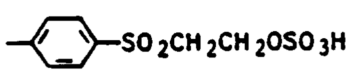 | 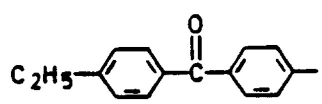 | blau-schwarz |

Tabelle 6

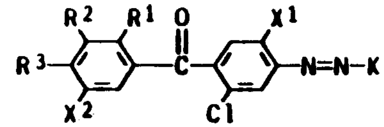

| Bsp. Nr. | K | $X^1$ | $R^1$ | $R^2$ | $R^3$ | $X^2$ | Farbton oder $\lambda_{max}$ [nm] |
|---|---|---|---|---|---|---|---|
| 65 | 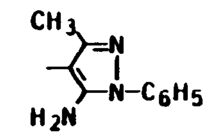 | H | H | $CH_3$ | $OCH_3$ | $SO_3H$ | gelb |
| 66 | | H | H | H | $OCH_3$ | $SO_3H$ | gelb |
| 67 | | H | H | H | $OCH_3$ | $SO_3H$ | gelb |
| 68 | | $SO_3H$ | H | H | $OCH_3$ | H | gelb |
| 69 | 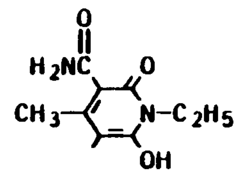 | $SO_3H$ | H | H | $OCH_3$ | H | grün-stichig gelb |
| 70 | | $SO_3H$ | H | H | $OCH_3$ | H | grün-stichig gelb |
| 71 | | $SO_3H$ | $CH_3$ | H | H | $CH_3$ | grün stichig gelb |
| 72 | 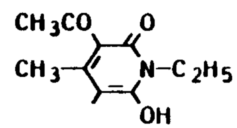 | $SO_3H$ | H | H | $CH_3$ | H | grün-stichig gelb |
| 73 | | H | $CH_3$ | H | $CH_3$ | $SO_3H$ | grün-stichig gelb |
| 74 | | H | H | H | $C_2H_5$ | $SO_3H$ | grün stichig gelb |

Tabelle 6 (Fortsetzung)

| Bsp. Nr. | K | $X^1$ | $R^1$ | $R^2$ | $R^3$ | $X^2$ | Farbton oder $\lambda_{max}$ [nm] |
|---|---|---|---|---|---|---|---|
| 75 | | H | H | H | $CH_3$ | H | grün-stichig gelb |
| 76 | | $SO_3H$ | H | H | $OCH_3$ | H | grün-stichig gelb |
| 77 | 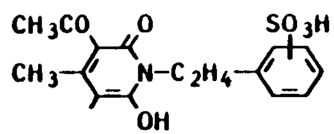 | $SO_3H$ | H | H | $C_2H_5$ | H | grün stichig |
| 78 | | $SO_3H$ | H | H | $CH(CH_3)_2$ | H | grün-stichig gelb |
| 79 | | H | H | H | $C_2H_5$ | $SO_3H$ | grün-stichig gelb |

| Bsp. Nr. | K | $X^1$ | $R^1$ | $R^2$ | $R^3$ | $X^2$ | Farbton oder $\lambda_{max}$ [nm] |
|---|---|---|---|---|---|---|---|
| 80 | 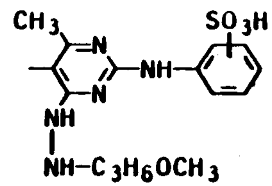 | $SO_3H$ | H | $CH_3$ | $CH_3$ | H | gelbstichig rot |
| 81 | | $SO_3H$ | H | H | $C_2H_5$ | H | gelbstichig rot |
| 82 | | $SO_3H$ | H | H | $CH(CH_3)_2$ | H | gelbstichig rot |
| 83 | 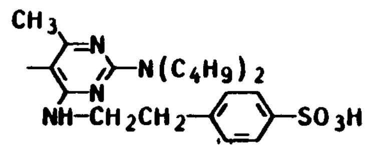 | $SO_3H$ | H | H | $C_3H_7(i)$ | H | orange |
| 84 | | $SO_3H$ | H | $CH_3$ | $CH_3$ | H | orange |
| 85 | | $SO_3H$ | H | H | $C_2H_5$ | H | orange |
| 86 | | $SO_3H$ | $CH_3$ | H | $CH_3$ | H | orange |
| 87 | 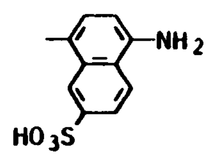 | $SO_3H$ | H | H | $C_3H_7(i)$ | H | 403 |
| 88 | | $SO_3H$ | H | $CH_3$ | $CH_3$ | H | 404 |
| 89 | 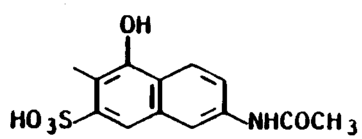 | $SO_3H$ | H | H | $C_2H_5$ | H | orange |
| 90 | | $SO_3H$ | $CH_3$ | H | $CH_3$ | H | orange |

| Bsp. Nr. | K | $X^1$ | $R^1$ | $R^2$ | $R^3$ | $X^2$ | Farbton oder $\lambda_{max}$ [nm] |
|---|---|---|---|---|---|---|---|
| 91 | 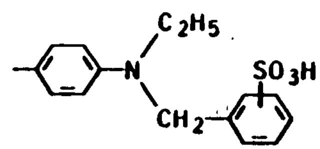 | $SO_3H$ | H | H | $CH_3$ | H | gelbstichig rot |
| 92 | | $SO_3H$ | $CH_3$ | H | $CH_3$ | H | gelbstichig rot |
| 93 | | $SO_3H$ | H | H | $C_2H_5$ | H | gelbstichig rot |
| 94 | 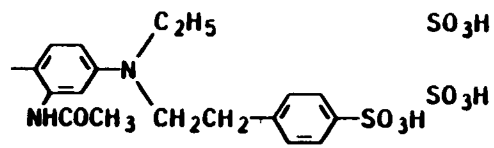 | $SO_3H$ | H | H | $CH_3$ | H | rot |
| 95 | | $SO_3H$ | H | H | $OCH_3$ | H | rot |
| 96 | | $SO_3H$ | $CH_3$ | H | H | $CH_3$ | rot |
| 97 | 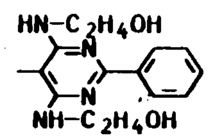 | $SO_3H$ | H | H | $OCH_3$ | H | orange |
| 98 | | $SO_3H$ | H | H | $CH_3$ | H | orange |

Tabelle 7

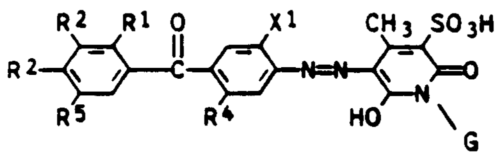

| Bsp. Nr. | $X^1$ | $R^1$ | $R^2$ | $R^3$ | $R^5$ | $R^4$ | G | $\lambda_{max}$ [nm] |
|---|---|---|---|---|---|---|---|---|
| 99 | H | H | H | $CH_3$ | H | H | $CH_3$ | 421 |
| 100 | H | H | H | $C_2H_5$ | H | H | $CH_3$ | 421 |
| 101 | H | H | $CH_3$ | $CH_3$ | H | H | $CH_3$ | 422 |
| 102 | H | $CH_3$ | H | $CH_3$ | H | H | $CH_3$ | 420 |
| 103 | H | $OCH_3$ | H | H | $CH_3$ | H | $CH_3$ | 422 |
| 104 | H | $CH_3$ | H | $OCH_3$ | H | H | $C_2H_5$ | 423 |
| 105 | H | H | H | $CH_3$ | H | Cl | $CH_3$ | 421 |
| 106 | H | H | H | $C_2H_5$ | H | Cl | $CH_3$ | 421 |
| 107 | H | H | H | $OCH_3$ | H | Cl | $CH_3$ | 422 |
| 108 | $SO_3H$ | H | $CH_3$ | $CH_3$ | H | H | $C_2H_5$ | |
| 109 | $SO_3H$ | $CH_3$ | H | $CH_3$ | H | H | $C_2H_5$ | |
| 110 | $SO_3H$ | H | H | $C_2H_5$ | H | H | $CH_3$ | |
| 111 | $SO_3H$ | H | H | $C_2H_5$ | H | Cl | $CH_3$ | |
| 112 | H | H | H | $C_2H_5$ | $SO_3H$ | H | $CH_3$ | 421 |
| 113 | H | H | H | $C_2H_5$ | $SO_3H$ | Cl | $CH_3$ | 421 |
| 114 | H | H | $CH_3$ | $CH_3$ | $SO_3H$ | H | $CH_3$ | 421 |
| 115 | H | H | $CH_3$ | $CH_3$ | $SO_3H$ | H | $C_4H_9(n)$ | 421 |
| 116 | H | H | $CH_3$ | $OCH_3$ | H | Cl | $CH_3$ | 422 |
| 117 | $SO_3H$ | H | $CH_3$ | $CH_3$ | H | $OCH_3$ | $CH_3$ | |
| 118 | H | H | H | $CH_3$ | H | $OCH_3$ | $CH_3$ | |

Beispiel 119

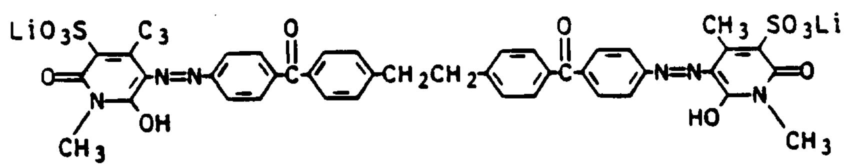

brillantes grünstichiges Gelb auf Leder, Polyamid und Wolle.

Beispiel 120

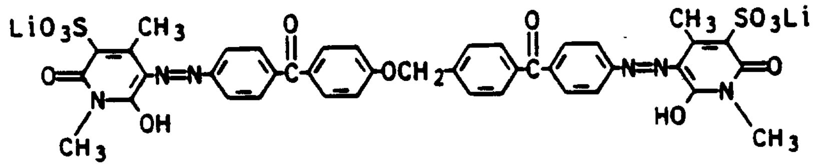

klares grünstichiges Gelb auf Leder, Polyamid und Wolle.

Beispiel 121

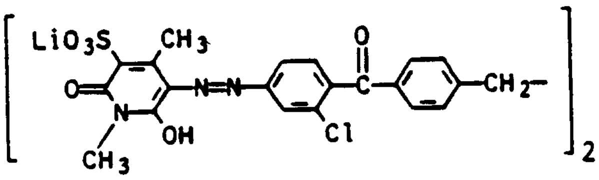

grünstichiges Gelb auf Leder, Polyamid und Wolle.

Beispiel 122

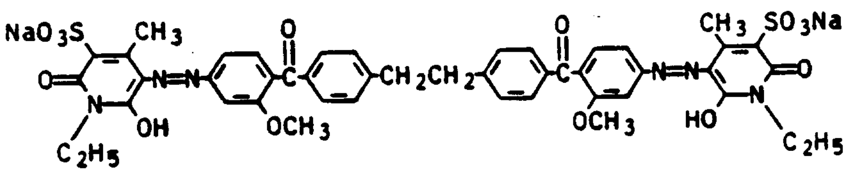

grünstichiges Gelb auf Polyamid, Wolle und Leder.

Beispiel 123

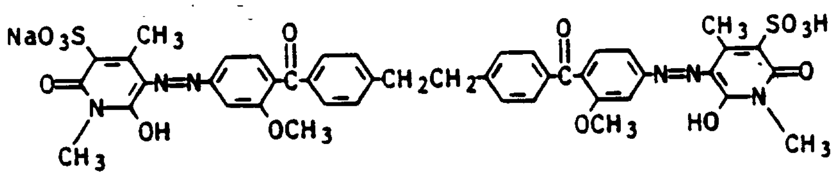

grünstichiges Gelb auf Leder und Wolle.

Beispiel 124

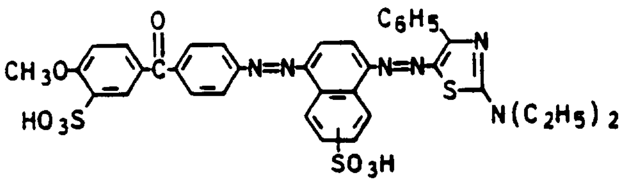

marineblau

Beispiel 125

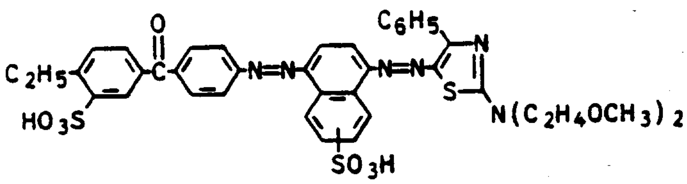

marineblau

Beispiel 126

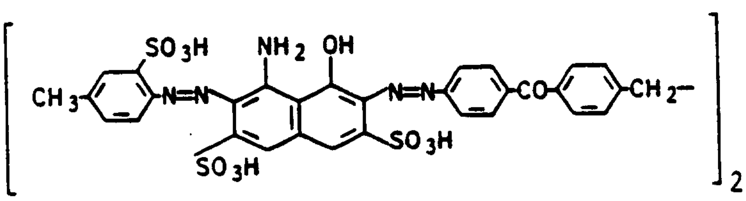

blau

Beispiel 127

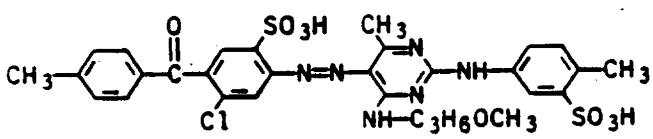

goldgelb

$\lambda_{max}$ ($H_2O$): 407 nm

Beispiel 128

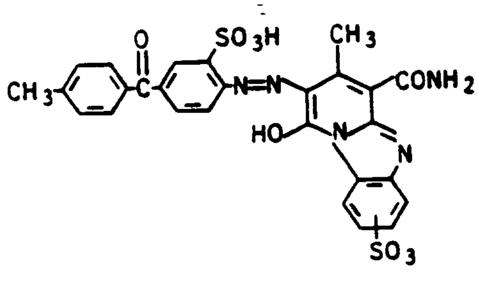

orange

Beispiel 129

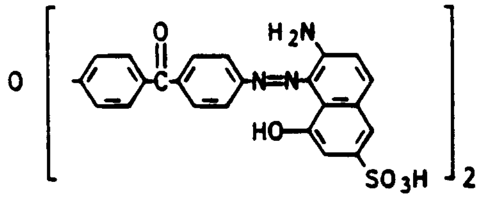

bordo

Beispiel 130

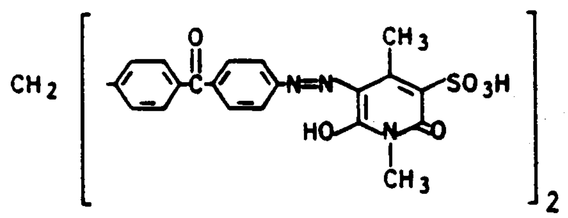

gelb

Beispiel 131

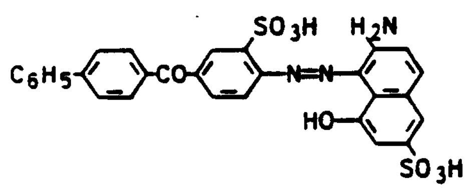

bordo

$\lambda_{max}$ ($H_2O$): 526 nm

Beispiel 132

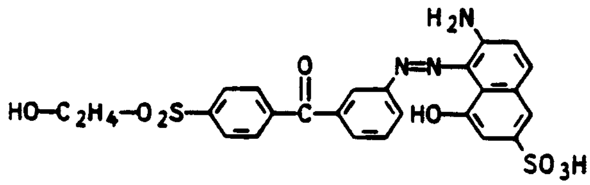

rot

Tabelle 8

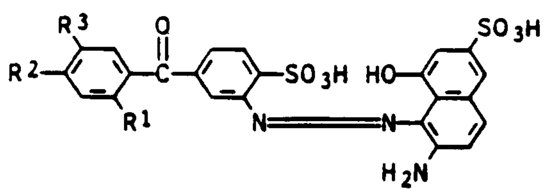

| Bsp. Nr. | R1 | R2 | R3 | Farbton | $\lambda_{max}$ ($H_2O$) [nm] |
|---|---|---|---|---|---|
| 133 | H | $CH_3$ | H | klares rot | 509 |
| 134 | H | $C_2H_5$ | H | klares rot | 509 |
| 135 | H | $CH(CH_3)_2$ | H | klares rot | 508 |
| 136 | $CH_3$ | $CH_3$ | H | klares rot | 509 |
| 137 | $CH_3$ | H | $CH_3$ | klares rot | 509 |
| 138 | H | $CH_3$ | $CH_3$ | klares rot | 509 |
| 139 | H | $C_6H_5$ | H | klares rot | 512 |
| 140 | H | Cl | H | klares rot | 511 |
| 141 | H | $CH_3$ | Cl | klares rot | 510 |

Beispiel 142 (Anwendung)

100 Gewichtsteile eines nachgegerbten Chromrindleders mit einer Falzstärke von 1,4 mm wurden in 200 Gewichtsteilen Wasser bei 30 °C mit 1 Gewichtsteil Natriumhydrogencarbonat und 1 Gewichtsteil Natriumformiat durch 45 minütiges Walken in einem Walkfaß neutralisiert. Das Leder wurde dann durch 15 minütiges Walken in 200 Gewichtsteilen frischem Wasser bei 30 °C gewaschen. Anschließend wurde die Färbung durch 45 minütiges Walken in 200 Gewichtstellen Wasser, das eine Temperatur von 50 °C aufwies und 1 Gew.-% des in Beispiel 2 beschriebenen Farbstoffs enthielt, vorgenommen. Dann wurden 4 Gewichtsteile eines handelsüblichen Fettlickers zugegeben und die Walke für 30 Minuten fortgesetzt. Nach dem Ansäuern mit 0,5 Gewichtsteilen Ameisensäure wurde das Leder weitere 30 Minuten gewalkt, dann mit Kaltem Wasser gespült, ausgereckt, getrocknet, gespänt, gestollt und gespannt.

Man erhielt ein in klarem, kräftigen zitronengelben Ton gefärbtes Leder. Die Färbung weist gute Lichtechtheit und gute Naßechtheiten auf.

Ähnliche günstige Ergebnisse bei der Färbung von Leder werden mit den im folgenden aufgeführten Farbstoffen erzielt.

Tabelle 9

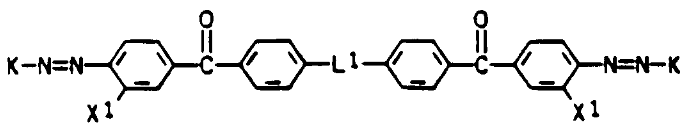

| Bsp. Nr. | $L^1$ | $X^1$ | K | Farbton |
|---|---|---|---|---|
| 143 | $CH_2CH_2$ | H | 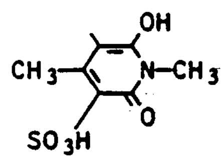 | grünstichig gelb |
| 144 | $CH_2CH_2$ | H | 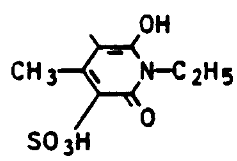 | grünstichig gelb |
| 145 | $CH_2CH_2$ | H | 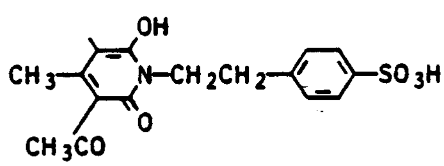 | grünstichig gelb |
| 146 | $CH_2CH_2$ | H | 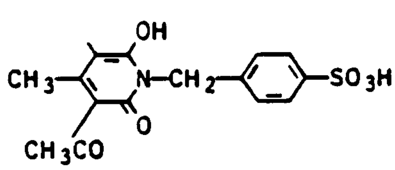 | grünstichig gelb |
| 147 | $CH_2CH_2$ | H | 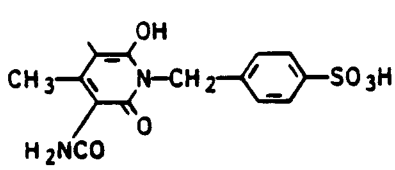 | grünstichig gelb |

Tabelle 9 (Fortsetzung)

| Bsp. Nr. | $L^1$ | $X^1$ | K | Farbton |
|---|---|---|---|---|
| 148 | $CH_2CH_2$ | H | 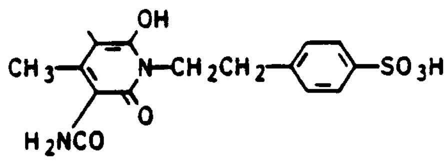 | grünstichig gelb |
| 149 | $CH_2CH_2$ | H | 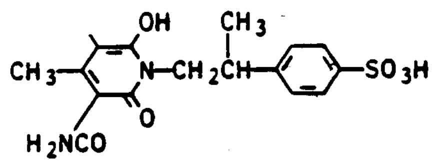 | grünstichig gelb |
| 150 | $CH_2$–O | H | 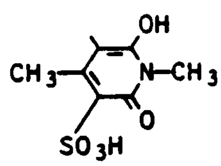 | grünstichig gelb |
| 151 | $CH_2CH_2$ | H | 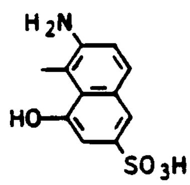 | blaustichig rot<br>$\lambda_{max}$ ($H_2O$): 529 nm |
| 152 | $CH_2CH_2$ | H | 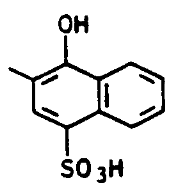 | orange |
| 153 | $CH_2CH_2$ | $SO_3H$ | 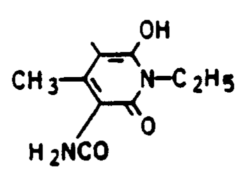 | grünstichig gelb |

Tabelle 9 (Fortsetzung)

| Bsp. Nr. | $L^1$ | $X^1$ | K | Farbton |
|---|---|---|---|---|
| 154 | $CH_2CH_2$ | $SO_3H$ | 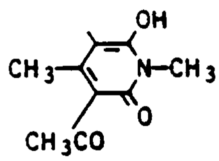 | grünstichig gelb |
| 155 | $CH_2CH_2$ | $SO_3H$ | 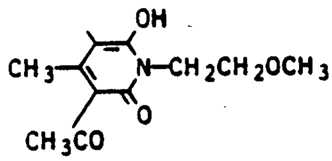 | grünstichig gelb |

Tabelle 10

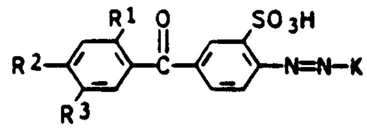

| Bsp. Nr. | $R^1$ | $R^2$ | $R^3$ | K | Farbton |
|---|---|---|---|---|---|
| 156 | H | $CH_3$ | H | 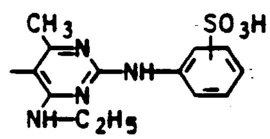 | rotorange |
| 157 | H | $CH_3$ | H | 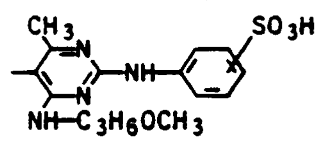 | rotorange |
| 158 | H | $C_2H_5$ | H | 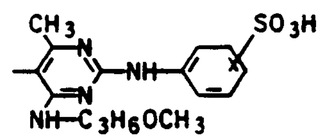 | rotorange |
| 159 | H | $CH_3$ | $CH_3$ | 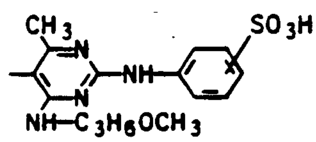 | rotorange |
| 160 | $CH_3$ | $CH_3$ | H | 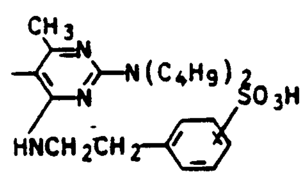 | orange |

Beispiel 161

42,1 g der Diazokomponente der Formel

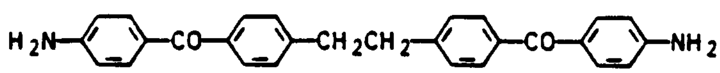

wurden bei Raumtemperatur mit 90 mi 25 gew.-%iger Salzsäure und 10 Tropfen eines sauer wirksamen Netzmittels verrührt. Nach 2 Stunden gab min 100 g Eis hinzu und versetzte die Suspension unter Kühlen mit insgesamt 64 ml 23 gew.-%iger wäßriger Natriumnitritlösung. Die Diazotierung rührte 2,5 Stunden bei 0 bis 8 °C nach. Dann zerstörte man überschüssige salpetrige Säure vollständig mit Amidosulfonsäure und verdünnte die erhaltene Diazoniumsalzlösung mit 150 ml Eiswasser. Das Gemisch lief dann zu 65,1 g der Kupplungskomponente der Formel

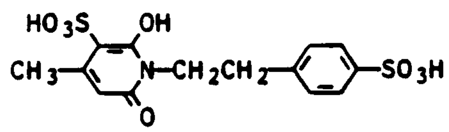

,

die bei pH 7 in 300 ml Wasser und 300 ml Eis gelöst worden waren. Während des Zulaufs der Diazoniumsalzlösung hielt man den pH-Wert des Reaktionsgemisches durch Zugabe von Natronlauge im Bereich von 4 bis 7. Die Kupplung war rasch beendet. Die erhaltene honiggelbe Farbstofflösung wurde bei einem pH-Wert von 4 bis 6 sprühgetrocknet. Man erhielt 122 g des Farbstoffes der Formel

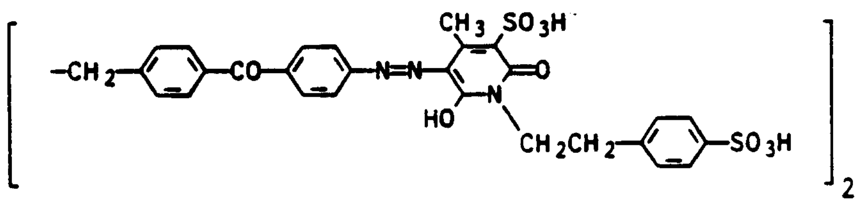

in Form des Natriumsalzes der Sulfonsäuren. Das gelbe Pulver enthält noch Natriumchlorid.

0,5 bis 1 %ige Färbungen dieses Farbstoffes auf nachgegerbtem Leder ergeben ein sehr klares, sehr gut naßechtes und lichtechtes grünstichiges Gelb. $\lambda_{max}$ ($H_2O$): 421 nm.

Verwendet man anstelle der oben genannten Diazokomponente eine Verbindung der Formel:

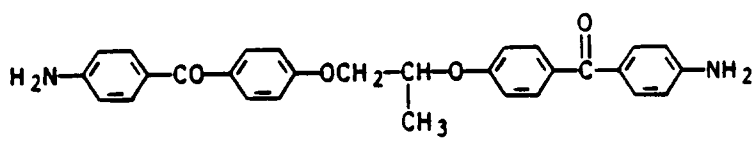

,

so erhält man einen gelben Farbstoff der Formel

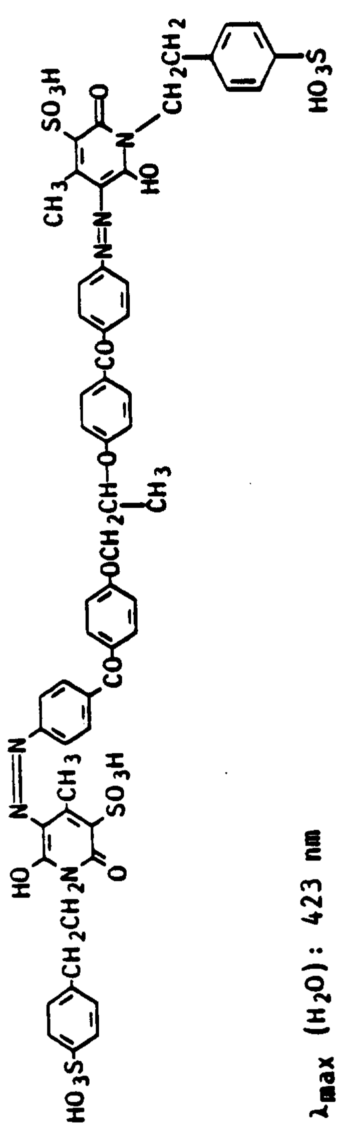

λmax ($H_2O$): 423 nm

der ein ähnliches Echtheitsprofil aufweist.

In analoger Weise werden die folgenden Verbindungen sowie die in den folgenden Tabellen 11 und 12 aufgeführten Farbstoffe erhalten.

Beispiel 162

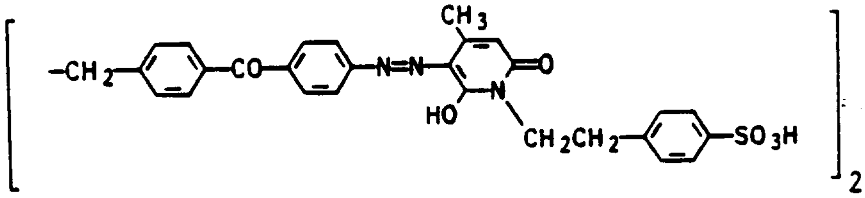

$\lambda_{max}$ ($H_2O$): 403 nm

Beispiel 163

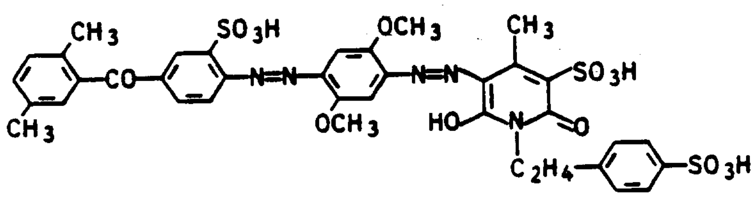

(Farbton: rot)

Tabelle 11

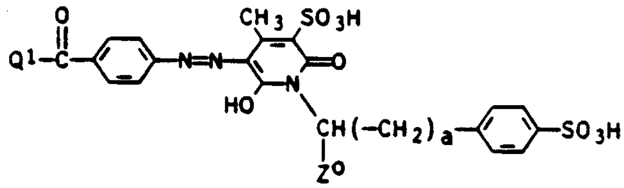

| Bsp. Nr. | $Q^1$ | $Z^0$ | a | $\lambda_{max}$ [nm] (in Wasser) | Farbton |
|---|---|---|---|---|---|
| 164 | 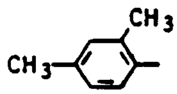 | H | 1 | 431 | gelb |
| 165 | 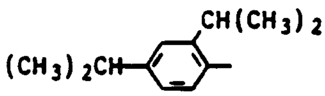 | H | 1 | 431 | gelb |
| 166 | 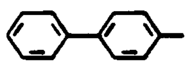 | H | 1 | | gelb |
| 167 | 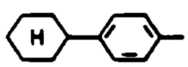 | H | 1 | 431 | gelb |
| 168 | 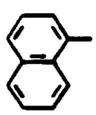 | H | 1 | | gelb |
| 169 | 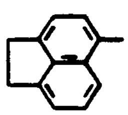 | H | 1 | | gelb |
| 170 | $C_6H_5-$ | H | 1 | | grünstichig gelb |
| 171 | 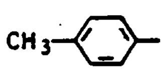 | H | 1 | 431 | grünstichig gelb |
| 172 | 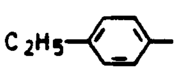 | H | 1 | 432 | grünstichig gelb |
| 173 | 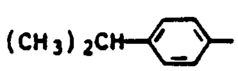 | H | 1 | 432 | grünstichig gelb |

Tabelle 11 (Fortsetzung)

| Bsp. Nr. | $Q^1$ | $Z^o$ | a | $\lambda_{max}$ [nm] (in Wasser) | Farbton |
|---|---|---|---|---|---|
| 174 | 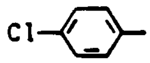 | H | 1 | 429 | grünstichig gelb |
| 175 | 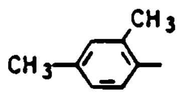 | H | 0 | 430 | grünstichig gelb |
| 176 | 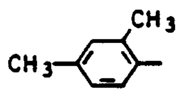 | $CH_3$ | 1 | 431 | grünstichig gelb |
| 177 | 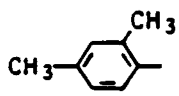 | $CH_3$ | 2 | 431 | grünstichig gelb |
| 178 | 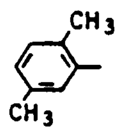 | H | 0 | 430 | grünstichig gelb |
| 179 | 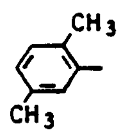 | H | 1 | 430 | grünstichig gelb |
| 180 | 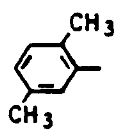 | $CH_3$ | 1 | 431 | grünstichig gelb |
| 181 | 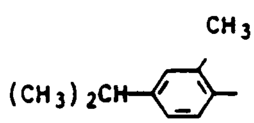 | H | 1 | 431 | grünstichig gelb |
| 182 | 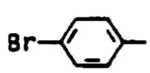 | H | 1 | 430 | grünstichig gelb |
| 183 | 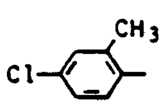 | H | 1 | 430 | grünstichig gelb |

Tabelle 11 (Fortsetzung)

| Bsp. Nr. | $Q^1$ | $Z^o$ | a | $\lambda_{max}$ [nm] (in Wasser) | Farbton |
|---|---|---|---|---|---|
| 184 | 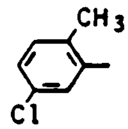 | H | 1 | 429 | grünstichig gelb |
| 185 | 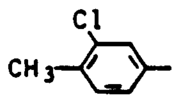 | H | 1 | 430 | grünstichig gelb |
| 186 | 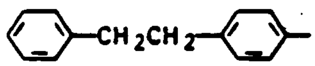 | H | 1 | 430 | grünstichig gelb |
| 187 | 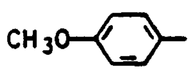 | H | 1 | 432 | gelb |
| 188 | 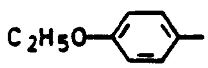 | H | 1 | 432 | gelb |
| 189 | 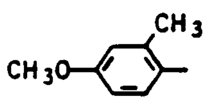 | H | 1 | 432 | gelb |
| 190 | 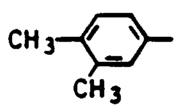 | H | 1 | 431 | grünstichig gelb |
| 191 | 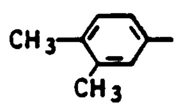 | $CH_3$ | 1 | 431 | grünstichig gelb |

Tabelle 12

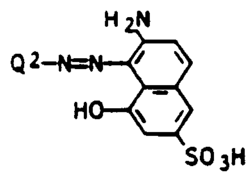

| Bsp. Nr. | $Q^2$ | $\lambda_{max}$ [nm] (in Wasser) | Farbton |
|---|---|---|---|
| 192 | 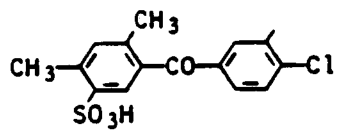 | | blaustichig rot |
| 193 | 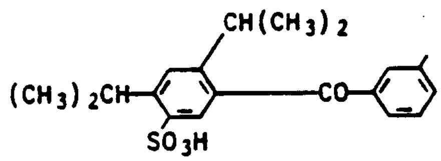 | | blaustichig rot |
| 194 | 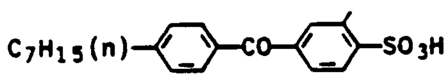 | 513 | blaustichig rot |
| 195 | 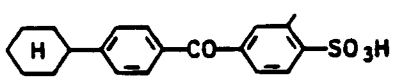 | 513 | blaustichig rot |
| 196 | 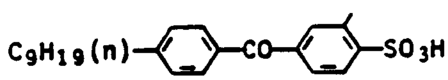 | 513 | blaustichig rot |
| 197 | 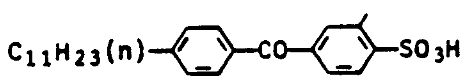 | 513 | blaustichig rot |
| 198 | 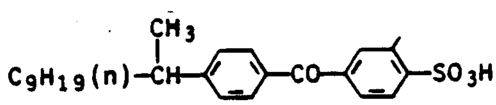 | 513 | blaustichig rot |
| 199 | 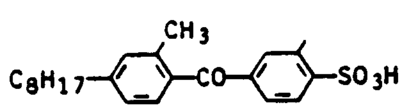 | 513 | blaustichig rot |

Tabelle 13

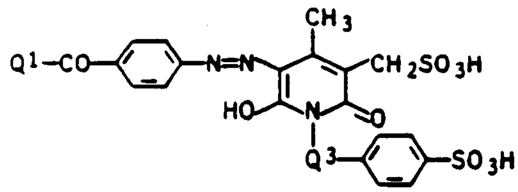

| Bsp. Nr. | $Q^1$ | $Q^3$ | Farbton |
|---|---|---|---|
| 200 | 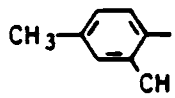 | $CH_2$ | grünstichig gelb |
| 201 | 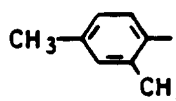 | $C_2H_4$ | grünstichig gelb |
| 202 | 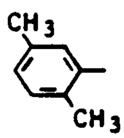 | $CH_2$ | grünstichig gelb |
| 203 | 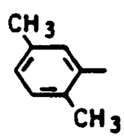 | $C_2H_4$ | grünstichig gelb |

Beispiel 204

257 g 1-(2-Phenylethyl)-6-hydroxy-3-cyano-4-methypyrid-2-on wurden bei maximal 70 °C in ein Gemisch aus 160 g 100 gew.-%ige Schwefelsäure und 380 g Oleum (24 gew.-%ig) eingetragen. Die entstandene Lösung wurde 5 Stunden bei 75 °C nachgerührt, wobei im wesentlichen ein Produkt der Formel

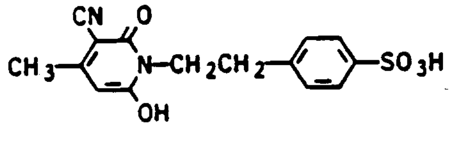

entstand. Dann erhitzte man innerhalb von 5 Stunden auf 110 °C, wobei die Cyanogruppe hydrolysierte und die intermediär entstehende Carbonsäure decarboxyliert wurde (Schäumen durch $CO_2$-Entwicklung). Anschließend wurde auf 130 bis 135 °C erhitzt und 5,5 Stunden bei dieser Temperatur gerührt. Nach Abkühlen des Reaktionsgemisches auf ca. 110 °C wurde auf ca. 900 g Eiswasser ausgertührt und die Verbindung der Formel

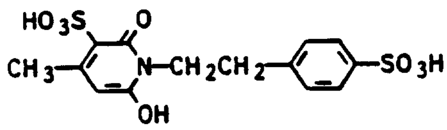

bei ≦ 30 °C mit Natronlauge neutralisiert. Diese Lösung kann direkt zur Herstellung von Azofarbstoffen verwendet werden.
$\lambda_{max}$ ($H_2O$): 325 nm, 247 nm, (Minimum bei 280 und 235 nm).

## Patentansprüche

**1.** Benzophenonazofarbstoffe der Formel I

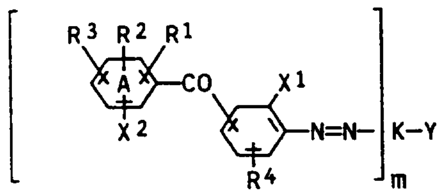

(I),

in der
der Ring A benzoanelliert sein und ein benzoanellierter Ring A durch $C_2H_4$ verbrückt sein kann,

m 1 oder 2,

K den Rest eines 6-Hydroxypyrid-2-onderivats, das in Ringposition 3 unsubstituiert oder durch Carbamoyl, $C_2$-$C_5$-Alkanoyl, Hydroxysulfonylmethyl oder Hydroxysulfonyl substituiert ist, den Rest einer Kupplungskomponente aus der Phenylazopyridon-, Imidazolopyridin-, Aminopyrazol-, Hydroxypyrazol-, Aminothiazol-, Pyrimidin-, Chinolon- oder Anilinreihe oder den Rest einer Kupplungskomponente der Formel IIm

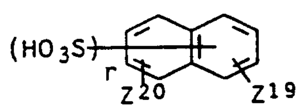

(IIm),

in der

$Z^{19}$ für Amino, Phenylamino, $C_1$-$C_4$-Alkanoylamino oder Benzoylamino,

$Z^{20}$ für Wasserstoff oder Hydroxy und

r für 1 stehen,

Y wenn m die Bedeutung von 1 besitzt, Wasserstoff oder den Rest -N=N-Q, worin Q für den Rest einer Diazo- oder Kupplungskomponente steht, oder, wenn m die Bedeutung von 2 besitzt, Wasserstoff, einer der beiden Reste $X^1$ und $X^2$ Wasserstoff und der andere Hydroxysulfonyl,

$R^1$, $R^2$ und $R^3$ gleich oder verschieden sind und unabhängig voneinander jeweils Wasserstoff, Halogen, $C_1$-$C_{12}$-Alkyl, Cyclohexyl, Phenyl, 2-Hydroxyethylsulfonyl oder $C_1$-$C_4$-Alkoxy oder, wenn m für 1 und Y für Wasserstoff stehen, einer dieser Substituenten auch den Rest der Formel

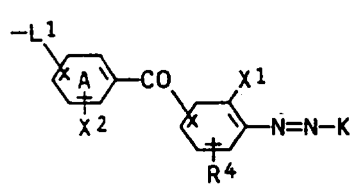

worin $L^1$ für eine chemische Bindung, $C_1$-$C_4$-Alkylen, Sauerstoff oder einen Rest der Formel O-$CH_2$, O-$CH_2CH_2$-O, O-$CH_2CH_2CH_2$-O oder O-$CH(CH_3)CH_2$-O steht und $X^1$, $X^2$, $R^4$, K und der Ring A jeweils die obengenannte Bedeutung besitzen, und

$R^4$ Wasserstoff, Halogen oder $C_1$-$C_4$-Alkoxy bedeuten.

**2.** Benzophenonazofarbstoffe nach Anspruch 1, die der Formel Ia

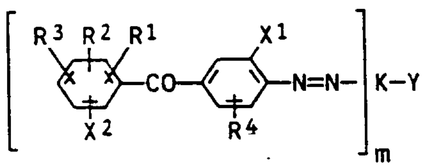

(Ia)

entsprechen, in der m, K, Y, $X^1$, $X^2$, $R^1$, $R^2$, $R^3$ und $R^4$ jeweils die obengenannte Bedeutung besitzen.

**3.** Benzophenonazofarbstoffe nach Anspruch 1, dadurch gekennzeichnet, daß K den Rest einer Kupplungskomponente aus der Pyridon-, Aminopyrazol- oder Pyrimidinreihe oder den Rest einer Kupplungskomponente der Formel IIm

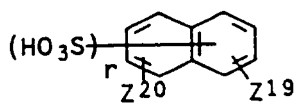

(IIm),

in der

$Z^{19}$ für Amino, Phenylamino, $C_1$-$C_4$-Alkanoylamino oder Benzoylamino,
$Z^{20}$ für Wasserstoff oder Hydroxy und
r für 1 stehen, bedeutet.

**4.** Verwendung der Benzophenonazofarbstoffe gemäß Anspruch 1 zum Färben von natürlichen oder synthetischen Substraten.

**5.** Pyridonverbindungen der Formel II

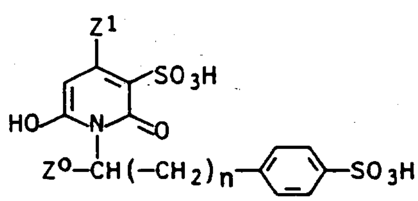

(II),

in der

n 1 oder 2,
$Z^o$ Wasserstoff oder $C_1$-$C_4$-Alkyl und
$Z^1$ Wasserstoff, $C_1$-$C_4$-Alkyl oder Phenyl bedeuten.

**6.** Verfahren zur Herstellung der Pyridonverbindungen der Formel IIa

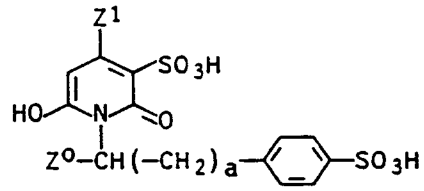

(IIa),

in der

- a 0, 1 oder 2,
- $Z^o$ Wasserstoff oder $C_1$-$C_4$-Alkyl und
- $Z^1$ Wasserstoff, $C_1$-$C_4$-Alkyl oder Phenyl bedeuten,

durch Umsetzung von Pyridonen der Formel IIb

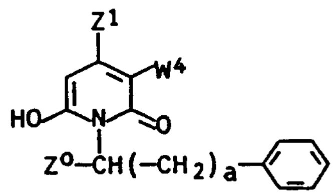

(IIb),

in der $W^4$ Cyano oder Acetyl bedeutet und a, $Z^o$ und $Z^1$ jeweils die obengenannte Bedeutung besitzen, mit Oleum, dadurch gekennzeichnet, daß man Oleum verwendet, das 1 bis 2 mol freies Schwefeltrioxid enthält, und man die Umsetzung in einer 1. Stufe 1 bis 5 Stunden bei einer Temperatur von 0 bis 75 °C und anschließend in einer 2. Stufe 2 bis 11 Stunden bei einer Temperatur von 80 bis 135 °C durchführt.

## Claims

**1.** A benzophenoneazo dye of the formula I

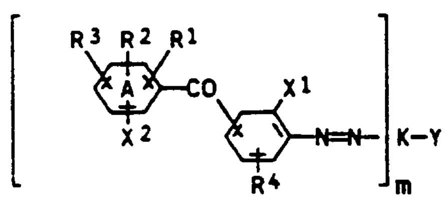

(I)

where

the ring A can be benzofused and a benzofused ring A can be bridged by $C_2H_4$,

- m is 1 or 2,
- K is the radical of a 6-hydroxyprid-2-one derivative which in ring position 3 is unsubstituted or substituted by carbamoyl, $C_2$-$C_5$-alkanoyl, hydroxysulfonylmethyl or hydroxysulfonyl, the radical of a coupling component of the phenylazopyridone, imidazolopyridine, aminopyrazole, hydroxypyrazole, aminothiazole, pyrimidine, quinolone or aniline series, or the radical of a coupling component of the formula IIm

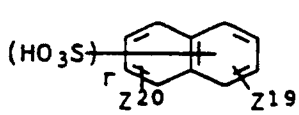

(IIm)

where

$Z^{19}$ is amino, phenylamino, $C_1$-$C_4$-alkanoylamino or benzoylamino,

$Z^{20}$ is hydrogen or hydroxyl, and

r is 1,

Y, when m is 1, is hydrogen or the radical -N=N-Q, where Q is the radical of a diazo or coupling component, or, when m is 2, is hydrogen,

one of the two radicals $X^1$ and $X^2$ is hydrogen and the other is hydroxysulfonyl,

$R^1$, $R^2$ and $R^3$ are identical or different and each is independently of the others hydrogen, halogen, $C_1$-$C_{12}$-alkyl, cyclohexyl, phenyl, 2-hydroxyethylsulfonyl or $C_1$-$C_4$-alkoxy or, when m is 1 and Y is hydrogen, one of these substituents can also be the radical of the formula

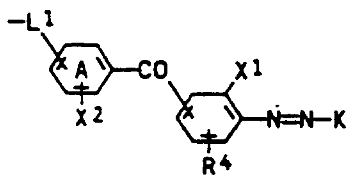

where $L^1$ is a chemical bond, $C_1$-$C_4$-alkylene, oxygen or a radical of the formula $O\text{-}CH_2$, $O\text{-}CH_2CH_2\text{-}O$, $O\text{-}CH_2CH_2CH_2\text{-}O$ or $O\text{-}CH(CH_3)CH_2\text{-}O$, and $X^1$, $X^2$, $R^4$, K and the ring A are each as defined above, and

$R^4$ is hydrogen, halogen or $C_1$-$C_4$-alkoxy.

**2.** A benzophenoneazo dye as claimed in claim 1 of the formula Ia

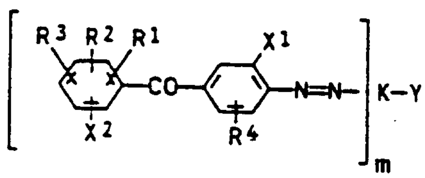

(Ia)

where m, K, Y, $X^1$, $X^2$, $R^1$, $R^2$, $R^3$ and $R^4$ are each as defined above.

**3.** A benzophenoneazo dye as claimed in claim 1, wherein K is the radical of a coupling component of the pyridone, aminopyrazole or pyrimidine series or the radical of a coupling component of the formula IIm

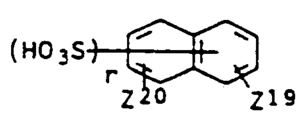

(IIm)

where

$Z^{19}$ is amino, phenylamino, $C_1$-$C_4$-alkanoylamino or benzoylamino,

$Z^{20}$ is hydrogen or hydroxyl, and

r is 1.

**4.** The use of a benzophenoneazo dye as claimed in claim 1 for dyeing natural or synthetic substrates.

**5.** A pyridone compound of the formula II

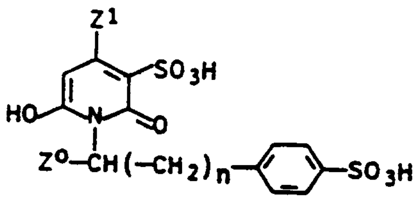

(II)

where

n is 1 or 2,

$Z^o$ is hydrogen or $C_1$-$C_4$-alkyl, and

$Z^1$ is hydrogen, $C_1$-$C_4$-alkyl or phenyl.

**6.** A process for preparing a pyridone compound of the formula IIa

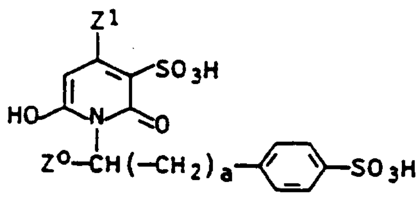

(IIa)

where

a is 0, 1 or 2,

$Z^o$ is hydrogen or $C_1$-$C_4$-alkyl, and

$Z^1$ is hydrogen, $C_1$-$C_4$-alkyl or phenyl,

by reacting a pyridone of the formula IIb

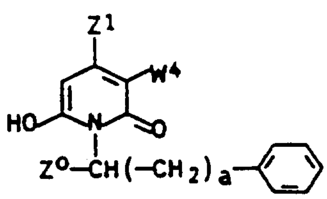

(IIb)

where $W^4$ is cyano or acetyl and a, $Z^o$ and $Z^1$ are each as defined above, with oleum, which comprises using oleum which contains from 1 to 2 mol of free sulfur trioxide and carrying out the reaction in a first stage at from 0 to 75 °C for from 1 to 5 hours and then in a second stage at from 80 to 135 °C for from 2 to 11 hours.

## Revendications

**1.** Colorants azoïques de la série de la benzophénone de formule I

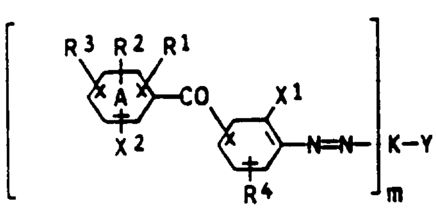

(I),

dans laquelle
le cycle A peut être benzocondensé et un cycle A benzocondensé peut être ponté par $C_2H_4$,

m est 1 ou 2,

K représente le reste d'un dérivé de 6-hydroxypyrid-2-one qui, en position 3 du cycle, n'est pas substitué ou est substitué par un groupe carbamoyle, alcanoyle en $C_2$-$C_5$, hydroxysulfonylméthyle ou hydroxysulfonyle, le reste d'un composant de couplage de la série des phénylazopyridone, imidazolopyridine, aminopyrazole, hydroxypyrazole aminothiazole, pyrimidine, quinolone ou aniline ou le reste d'un composant de couplage de formule IIm

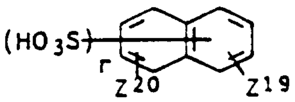

(IIm),

dans laquelle

$Z^{19}$ est mis pour un groupe amino, phénylamino, aloanoylamino en $C_1$-$C_4$ ou benzoylamino,

$Z^{20}$ est mis pour un atome d'hydrogéne ou un groupe hydroxy et

r est 1,

Y représente, lorsque m est 1, un atome d'hydrogène ou le reste -N=N-Q, dans lequel Q est le reste d'un composent diazoïque ou de couplage, ou, lorsque m est 2, un atome d'hydrogène,

l'un des deux restes $X^1$ et $X^2$ étant un atome d'hydrogène et l'autre un groupe hydroxysulfonyle,

$R^1$, $R^2$ et $R^3$ sont identiques ou différents et représentent chacun indépendamment l'un de l'autre un atome d'hydrogène, d'halogène, un groupe alkyle en $C_1$-$C_{12}$, cyclohexyle, phényle, 2-hydroxyéthyl-sulfonyle ou alcoxy en $C_1$-$C_4$ ou, lorsque m est 1 et Y est un atome d'hydrogène, l'un de ces substituants peut également représenter le reste de formule

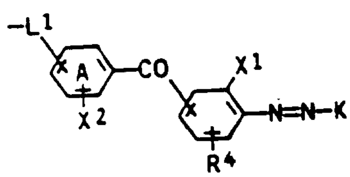

dans laquelle $L^1$ est mis pour une liaison chimique, un groupe alkylène en $C_1$-$C_4$, un atome d' oxygène ou un reste de formule O-$CH_2$, O-$CH_2CH_2$-O, O-$CH_2CH_2CH_2$-O ou O-CH($CH_3$)$CH_2$-O et $X^1$, $X^2$, $R^4$, K et le cycle A ont chaque fois la signification indiquée ci-dessus, et

$R^4$ représente un atome d'hydrogène, d'halogène ou un groupe alcoxy en $C_1$-$C_4$.

2. Colorants azoïques de la série de la benzophénone selon la revendication 1, qui ont la formule Ia

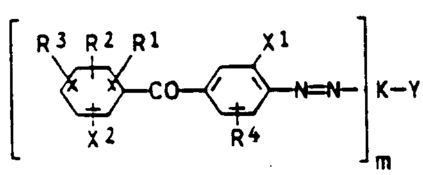

(Ia)

dans laquelle m, K, Y, $X^1$, $X^2$, $R^1$, $R^2$, $R^3$ et $R^4$ ont chaque fois la signification indiquée ci-dessus.

3. Colorants azoïques de la série de la benzophénone selon la revendication 1, carectérisés en ce que K représente le reste d'un composent de couplage de la série des pyridone, aminopyrazole ou pyrimidine ou le reste d'un composent de couplage de formule IIm

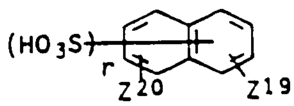

(IIm),

dans laquelle

- $Z^{19}$ est mis pour un groupe amino, phénylamino, alcanoylamino en $C_1$-$C_4$ ou benzoylamino,
- $Z^{20}$ est mis pour un atome d'hydrogène ou un groupe hydroxy et
- r est 1.

**4.** Utilisation des colorants azoïques de la série de la benzophénone selon la revendication 1 pour teindre des substrats naturels ou synthétiques.

**5.** Composés de pyridone de formule II

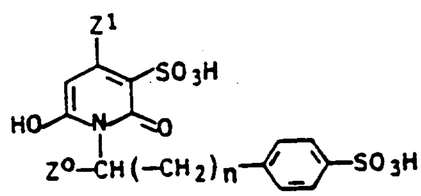

(II),

dans laquelle

- n est 1 ou 2,
- $Z^o$ est un atome d'hydrogène ou un groupe alkyle en $C_1$-$C_4$ et
- $Z^1$ est un atome d'hydrogène, un groupe alkyle en $C_1$-$C_4$ ou phényle.

**6.** Procédé de préparation des composes de pyridone de formule IIa

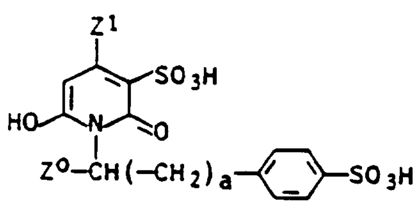

(IIa),

dans laquelle

- a est 0, 1 ou 2,
- $Z^o$ est un atome d'hydrogène ou un groupe alkyle en $C_1$-$C_4$ et
- $Z^1$ est un atome d'hydrogène, un groupe alkyle en $C_1$-$C_4$ ou phényle,

par réaction de pyridones de formule IIb

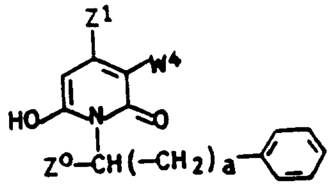

(IIb),

dans laqujelle $W^4$ représente un groupe cyano ou acétyle et a, $Z^o$ et $Z^1$ ont chaque fois la signification indiquée ci-dessus, avec de l'oléum, caractérisé en ce que l'on utilise de l'oléum qui contient 1 a 2 moles de trioxyde de soufre libre, et en ce que l'on effectue la réaction dans une première étape pendant 1 a 5 h à une température comprise entre 0 et 75 ° C, puis dans une seconde étape, pendant 2 à 11 h à une température comprise entre 80 et 135 ° C.